(19) 

Europäisches
Patentamt
European
Patent Office
Office européen
des brevets

(11)  **EP 4 140 953 A1**

(12)  **EUROPEAN PATENT APPLICATION**

(43) Date of publication:
**01.03.2023  Bulletin 2023/09**

(21) Application number: **21193840.2**

(22) Date of filing: **30.08.2021**

(51) International Patent Classification (IPC):
**C01F 5/24** *(2006.01)*       **C09C 1/02** *(2006.01)*
**A23L 29/00** *(2016.01)*       **A61K 8/00** *(2006.01)*
**A61K 33/10** *(2006.01)*

(52) Cooperative Patent Classification (CPC):
**C09C 1/028; A23L 29/206; A23L 33/16;**
**A61K 8/00; A61K 33/10; C01F 5/24;**
C01P 2004/03; C01P 2004/32; C01P 2004/50;
C01P 2004/51; C01P 2004/61; C01P 2006/10;
C01P 2006/12; C01P 2006/14; C01P 2006/19;

(Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Omya International AG**
**4665 Oftringen (CH)**

(72) Inventors:
• **MAURER, Marc**
  **68128 Village-Neuf (FR)**

• **CREMASCHI, Alain**
  **51240 Saint Germain La Ville (FR)**
• **KARL, Francine**
  **51510 Compertrix (FR)**
• **IVANOV, Julia**
  **4102 Binningen (CH)**
• **HILTY-VANCURA, Florentine Marianne**
  **8706 Meilen (CH)**
• **SCHLOTTERBACH, Thomas**
  **9500 Villach (AT)**

(74) Representative: **Maiwald GmbH**
**Elisenhof**
**Elisenstraße 3**
**80335 München (DE)**

(54)  **METHOD FOR THE PRODUCTION OF GRANULES COMPRISING A MAGNESIUM ION-COMPRISING MATERIAL**

(57)    The present invention refers to a method for the production of granules comprising a magnesium ion-comprising material, granules comprising the magnesium ion-comprising material and the use of the granules in a nutraceutical product, agricultural product, veterinary product, cosmetic product, preferably in a dry cosmetic and/or dry skin care composition, home product, food product, packaging product, personal care product, preferably in an oral care composition in air treatment and in water treatment, or as excipient in a pharmaceutical product.

EP 4 140 953 A1

(52) Cooperative Patent Classification (CPC): (Cont.)
C01P 2006/21; C01P 2006/22

**Description**

The present invention refers to a method for the production of granules comprising a magnesium ion-comprising material, granules comprising the magnesium ion-comprising material and the use of the granules in a nutraceutical product, agricultural product, veterinary product, cosmetic product, preferably in a dry cosmetic and/or dry skin care composition, home product, food product, packaging product, personal care product, preferably in an oral care composition, in air treatment and in water treatment, or as excipient in a pharmaceutical product.

In many applications such as pharmaceutical, nutraceutical, agricultural, veterinary, cosmetic, home, food, packaging, personal care product, in air treatment and in water treatment granules are of considerable importance and more preferred than powders. Thus, agglomeration of powders leading to granules typically having a size range between 0.2 to 4.0 mm depending on their subsequent use is widely used to improve physical properties of powders like wettability, flowability, bulk density and product appearance.

Furthermore, granulation is carried out, e.g. to prevent the segregation of the constituents of powder mixes, to prevent dusting or to improve flowability.

Granulation, i.e. the process in which the primary powder particles are made to adhere to form larger, multiparticle entities is a process of collecting particles together by creating bonds between them e.g. by a binding agent.

One of the most important types of granulation is wet granulation, wherein granules are formed by the addition of a granulation liquid onto a powder bed which is under the influence of an impeller. The agitation resulting in the system along with the wetting of the components within the formulation results in the agglomeration of the primary powder particles to produce wet granules. The granulation liquid contains a solvent which must be volatile so that it can be removed by drying, and be non-toxic. Water mixed into the powders can form bonds between powder particles that are strong enough to lock them together. However, once the water dries, the agglomerates may fall apart. Therefore, water may not be strong enough to create and hold a bond. In such instances, the granulation liquid includes a binder.

Regarding surface-reacted magnesium carbonate, also granules are generally known. For example, in EP3733785 A1, it is mentioned that the surface-reacted magnesium carbonate obtained from the process described therein may be part of a delivery system which may be in the form of a free flowing powder, a tablet, a pellet, or granules. Similarly, EP3517502 A1 refers to a delivery system for the release of one or more active agent(s) in a home care formulation comprising a carrier material consisting of magnesium carbonate having a specific surface area of $\geq 25$ $m^2/g$, measured using nitrogen and the BET method according to ISO 9277:2010, wherein the delivery system may be in the form of a powder, a tablet, a pellet, or granules.

However, granules which are obtained by the processes of the prior art such as by (wet) granulation, suffer from several disadvantages, such as e.g. inferior bulk density, flow properties and compactability as well as low mechanical stability. Furthermore, wet granulation requires the use of a binder which is less advantageous.

Thus, magnesium ion-comprising materials can be granulated using various methods, but the conventional processes, in the absence of binder, do not provide the desired result, i.e. high bulk density, flow properties and compactability as well as high mechanical stability.

Thus, it is the object of the present invention to provide a method for the production of granules comprising a magnesium ion-comprising material having a high bulk density, flow properties and compactability as well as high mechanical stability. A further object of the present invention is to improve the aforementioned characteristics without the use of a binder.

One or more of the foregoing and other objects are solved by the subject-matter as defined herein in the independent claim. Advantageous embodiments of the present invention are defined in the corresponding sub-claims.

The present invention thus relates to a method for the production of granules comprising a magnesium ion-comprising material, the method comprising the steps of

a) providing an aqueous suspension comprising a magnesium ion-comprising material;
b) homogenizing the aqueous suspension comprising a magnesium ion-comprising material of step a), and
c) removing the liquid from the aqueous suspension comprising a magnesium ion-comprising material of step b) by means of spray drying for obtaining granules comprising a magnesium ion-comprising material.

According to one embodiment, the magnesium ion-comprising material of step a) is selected from the group comprising a magnesium hydroxide-comprising material, a magnesium carbonate-comprising material, a magnesium oxide-comprising material and mixtures thereof, preferably the magnesium ion-comprising material of step a) is a magnesium carbonate-comprising material selected from the group consisting of dolomite ($CaMg(CO_3)_2$), anhydrous magnesium carbonate or magnesite ($MgCO_3$), hydromagnesite ($Mg_5(CO_3)_4(OH)_2 \cdot 4H_2O$), artinite ($Mg_2(CO_3)(OH)_2 \cdot 3H_2O$), dypingite ($Mg_5(CO_3)_4(OH)_2 \cdot 5H_2O$), giorgiosite ($Mg_5(CO_3)_4(OH)_2 \cdot 5H_2O$), pokrovskite ($Mg_2(CO_3)(OH)_2 \cdot 0.5H_2O$), barringtonite ($MgCO_3 \cdot 2H_2O$), lansfordite ($MgCO_3 \cdot 5H_2O$), dolocarbonate and nesquehonite ($MgCO_3 \cdot 3H_2O$), more preferably the magnesium ion-comprising material of step a) is hydromagnesite, e.g. natural or synthetic hydromagnesite.

According to another embodiment, the magnesium ion-comprising material of step a) is a surface-reacted magnesium carbonate-comprising material obtained by treating the surface of the magnesium carbonate-comprising material with one or more compound(s) selected from the group consisting of sulphuric acid, phosphoric acid, carbonic acid, carboxylic acids containing up to six carbon atoms, preferably selected from formic acid, acetic acid, propionic acid, lactic acid and mixtures thereof; and di-, and tri-carboxylic acids where the carboxylic acid groups are linked by a chain of 0-4 intermittent carbon atoms, preferably selected from oxalic acid, citric acid, succinic acid, maleic acid, malonic acid, tartaric acid, adipic acid, fumaric acid and mixtures thereof, or a corresponding salt thereof.

According to yet another embodiment, the magnesium ion-comprising material of step a) has

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and/or

b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and/or

c) a BET specific surface area in the range from 10 to 100 $m^2$/g, preferably from 12 to 70 $m^2$/g, and most preferably from 17 to 60 $m^2$/g, measured using nitrogen and the BET method according to ISO 9277:2010; and/or

d) an intra-particle intruded specific pore volume in the range from 0.9 to 2.3 $cm^3$/g, preferably from 1.2 to 2.1 $cm^3$/g, and most preferably from 1.5 to 2.0 $cm^3$/g, calculated from mercury porosimetry measurement.

According to one embodiment, the aqueous suspension of step a) has a solids content in the range from 1 to 40 wt.-%, preferably from 5 to 35 wt.-%, and most preferably from 7 to 26 wt.-%, based on the total weight of the aqueous suspension.

According to another embodiment, at least one disintegrant is added before and/or during and/or after step b), preferably the at least one disintegrant is selected from the group comprising sodium croscarmellose, modified cellulose gums, insoluble cross-linked polyvinylpyrrolidones, starches, modified starches, starch glycolates such as sodium starch glycolate, micro crystalline cellulose, pregelatinized starch, sodium carboxymethyl starch, low-substituted hydroxypropyl cellulose, homopolymers of N-vinyl-2-pyrrolidone, alkyl-, hydroxyalkyl-, carboxyalkyl-cellulose esters, alginic acid, microcrystalline cellulose and its polymorphic forms, ion exchange resins, gums, chitin, chitosan, clays, gellan gum, crosslinked polacrillin copolymers, agar, gelatine, dextrines, acrylic acid polymers, carboxymethylcellulose sodium/calcium, hydroxpropyl methyl cellulose phthalate, shellac, effervescent mixtures such as bicarbonates in combination with one or more acids, e.g. citric acid or tartaric acid, or mixtures thereof.

According to yet another embodiment, the at least one disintegrant is added before and/or during and/or after step b) in an amount ranging from 0.3 to 10 wt.-%, preferably from 0.5 to 8 wt.-%, more preferably from 1 to about 5 wt.-% based on the total dry weight of the magnesium ion-comprising material.

According to one embodiment, the homogenizing in step b) is carried out once or several times, preferably 1 to 5 times, more preferably 1 to 3 times.

According to another embodiment, the homogenizing in step b) is carried out by milling.

According to yet another embodiment, the homogenizing in step b) is carried out at

a) a pressure ranging from 50 to 900 bar, preferably from 100 to 750 bar, and most preferably from 130 to 650 bar, and/or

b) an initial temperature ranging from 5 to 95°C, preferably from 10 to 80°C, and most preferably from 15 to 60°C.

According to one embodiment, the spray drying in step c) is carried out at

a) a feed pressure ranging from 0.1 to 300 bar, preferably from 1 to 100 bar, more preferably from 1 to < 50 bar, and most preferably from 1 to 25 bar, and/or

b) a temperature measured as inlet temperature ranging from 120 to 950°C, preferably from 175 to 700°C, and most preferably from 180 to 550°C.

According to a further aspect, granules are provided comprising a magnesium ion-comprising material, wherein the granules have a bulk density ranging from 0.10 to 0.70 g/mL, preferably from 0.12 to 0.65 g/mL, more preferably from 0.20 to 0.60 g/mL and most preferably from 0.15 to 0.50 g/mL.

According to one embodiment, the granules have

a) a volume particle size $d_{90}$ of from 15 to 500 $\mu$m, preferably from 20 to 400 $\mu$m, and most preferably from 30 to 250 $\mu$m, as measured dry at 0.1 bar dispersion pressure by laser diffraction, and

b) a volume median particle size $d_{50}$ of from 5 to 300 $\mu$m, preferably from 8 to 200 $\mu$m, and most preferably from 10 to 150 $\mu$m, as measured dry at 0.1 bar dispersion pressure by laser diffraction, and

c) a volume particle size $d_{10}$ of from 1 to 100 $\mu$m, preferably from 2 to 70 $\mu$m, and most preferably from 4 to 50 $\mu$m, as measured dry at 0.1 bar dispersion pressure by laser diffraction, and/or

d) a BET specific surface area in the range from 20 to 90 m$^2$/g, preferably from 30 to 80 m$^2$/g, and most preferably from 40 to 70 m$^2$/g, measured using nitrogen and the BET method according to ISO 9277:2010, and/or

e) a spherical shape.

According to another embodiment, the granules comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and/or

b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and/or

c) a BET specific surface area in the range from 10 to 100 m$^2$/g, preferably from 12 to 70 m$^2$/g, and most preferably from 17 to 60 m$^2$/g, measured using nitrogen and the BET method according to ISO 9277:2010; and/or

d) an intra-particle intruded specific pore volume in the range from 0.9 to 2.3 cm$^3$/g, preferably from 1.2 to 2.1 cm$^3$/g, and most preferably from 1.5 to 2.0 cm$^3$/g, calculated from mercury porosimetry measurement.

According to yet another embodiment, the granules are obtained by a method as defined herein.

According to another aspect, the use of granules as defined herein in a nutraceutical product, agricultural product, veterinary product, cosmetic product, preferably in a dry cosmetic and/or dry skin care composition, home product, food product, packaging product, personal care product, preferably in an oral care composition, in air treatment and in water treatment, or as excipient in a pharmaceutical product is provided.

It should be understood that for the purpose of the present invention the following terms have the following meaning.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

Terms like "obtainable" or "definable" and "obtained" or "defined" are used interchangeably.

This e.g. means that, unless the context clearly dictates otherwise, the term "obtained" does not mean to indicate that e.g. an embodiment must be obtained by e.g. the sequence of steps following the term "obtained" though such a limited understanding is always included by the terms "obtained" or "defined" as a preferred embodiment.

According to the present invention, the method for the production of granules comprising a magnesium ion-comprising material comprises the steps of

a) providing an aqueous suspension comprising a magnesium ion-comprising material;

b) homogenizing the aqueous suspension comprising a magnesium ion-comprising material of step a), and

c) removing the liquid from the aqueous suspension comprising a magnesium ion-comprising material of step b) by means of spray drying for obtaining granules comprising a magnesium ion-comprising material.

It has been especially found out that the method according to the present invention must comprise a step of homogenizing an aqueous suspension comprising a magnesium ion-comprising material for obtaining granules having a high bulk density, flow properties and compactability as well as high mechanical stability.

In the following, it is referred to further details of the present invention and especially the foregoing method for the production of granules comprising a magnesium ion-comprising material.

One requirement of the present invention is that according to step a), an aqueous suspension comprising a magnesium ion-comprising material is provided.

It is appreciated that the term "magnesium ion-comprising" refers to a material that comprises at least 38 wt.-% of a magnesium compound. In one embodiment, the magnesium ion-comprising material comprises at least 38 wt.-%, preferably between 38 and 100 wt.-%, more preferably between 38 and 99.95 wt.-%, e.g. from 38 to 55 wt.-%, based on the total dry weight of the material, of the magnesium compound. In another embodiment, the magnesium ion-

comprising material comprises at least 85 wt.-%, preferably between 85 and 100 wt.-%, more preferably between 90 and 99.95 wt.-%, based on the total dry weight of the material, of the magnesium compound. Thus, it is to be noted that the magnesium ion-comprising material may further comprise impurities typically associated with the type of material used. For example, the magnesium ion-comprising material may further comprise impurities such as calcium ion-comprising materials like calcium hydroxide, calcium carbonate and mixtures thereof.

For example, if the magnesium ion-comprising material comprises the magnesium compound in an amount of at least 38 wt.-%, preferably between 38 and 100 wt.-%, more preferably between 38 and 99.95 wt.-%, e.g. from 38 to 45 wt.-%, based on the total dry weight of the material, the impurities such as calcium ion-comprising materials like calcium hydroxide, calcium carbonate and mixtures thereof are present in amounts of less than 62 wt.-%, preferably between 0 and 62 wt.-%, more preferably between 0.05 and 62 wt.-%, e.g. from 45 to 62 wt.-%, based on the total dry weight of the material. If the magnesium ion-comprising material comprises the magnesium compound in an amount of at least 85 wt.-%, preferably between 85 and 100 wt.-%, more preferably between 90 and 99.95 wt.-%, based on the total dry weight of the material, the impurities such as calcium ion-comprising materials like calcium hydroxide, calcium carbonate and mixtures thereof are present in amounts of less than 15 wt.-% and most preferably from 0.05 to 10 wt.-%, based on the total dry weight of the material. It is further appreciated that the magnesium ion-comprising material may be a mineral phase comprising calcium and magnesium ions, such as dolomite ($MgCa(CO_3)_2$).

The magnesium ion-comprising material can be a naturally occurring or synthetic magnesium ion-comprising material.

According to one embodiment of the present invention the naturally occurring magnesium ion-containing material may be obtained by dry grinding. According to another embodiment of the present invention, the naturally occurring magnesium ion-comprising material may be obtained by wet grinding and optionally subsequent drying.

In general, the grinding step can be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the magnesium ion-comprising material is obtained by wet-grinding, the grinding step may be performed under conditions such that autogenous grinding takes place and/or by horizontal ball milling, and/or other such processes known to the skilled man. The wet processed ground magnesium ion-comprising material thus obtained may be washed and dewatered by well-known processes, e.g. by flocculation, filtration or forced evaporation prior to drying. The subsequent step of drying may be carried out in a single step such as spray drying, or in at least two steps. It is also common that such a mineral material undergoes a beneficiation step (such as a flotation, bleaching or magnetic separation step) to remove impurities.

Synthetic magnesium ion-comprising materials in the meaning of the present invention can be obtained by processes well known in the art. For instance, US1361324, US935418, GB548197 and GB544907 generally describe the formation of aqueous solutions of magnesium bicarbonate (typically described as "$Mg(HCO_3)_2$"), which is then transformed by the action of a base, e.g., magnesium hydroxide, to form hydromagnesite. Other processes described in the art suggest to prepare compositions containing both, hydromagnesite and magnesium hydroxide, wherein magnesium hydroxide is mixed with water to form a suspension which is further contacted with carbon dioxide and an aqueous basic solution to form the corresponding mixture; cf. for example US5979461. EP0526121 describes a calcium-magnesium carbonate composite consisting of calcium carbonate and magnesium carbonate hydroxide and a method for the preparation thereof. Furthermore, GB594262 relates to a method and apparatus for treating magnesia-containing materials, such as magnesium and calcium carbonate materials for obtaining respective carbonates in discrete and separate forms, by controlled carbonation such that the magnesium and calcium carbonates may be separated by mechanical means and with attainment of special utilities in separated products. US2007194276 describes a method of reductively bleaching a mineral slurry comprising adding in the mineral slurry an effective amount of a formamidine sulfinic acid (FAS) and an effective amount of a borohydride to reductively bleach the mineral slurry.

For example, the magnesium ion-comprising material encompasses a naturally occurring or synthetic magnesium ion-comprising material.

In particular, the magnesium ion-comprising material of step a) is selected from the group comprising a magnesium hydroxide-comprising material, a magnesium carbonate-comprising material, a magnesium oxide-comprising material and mixtures thereof. Preferably, the magnesium ion-comprising material of step a) is a magnesium carbonate-comprising material.

Thus, the magnesium ion-comprising material of step a) may be selected from the group comprising brucite ($Mg(OH)_2$), periclase (MgO), dolomite ($CaMg(CO_3)_2$), anhydrous magnesium carbonate or magnesite ($MgCO_3$), hydromagnesite ($Mg_5(CO_3)_4(OH)_2 \cdot 4H_2O$), artinite ($Mg_2(CO_3)(OH)_2 \cdot 3H_2O$), dypingite ($Mg_5(CO_3)_4(OH)_2 \cdot 5H_2O$), giorgiosite ($Mg_5(CO_3)_4(OH)_2 \cdot 5H_2O$), pokrovskite ($Mg_2(CO_3)(OH)_2 \cdot 0.5H_2O$), barringtonite ($MgCO_3 \cdot 2H_2O$), lansfordite ($MgCO_3 \cdot 5H_2O$), dolocarbonate and nesquehonite ($MgCO_3 \cdot 3H_2O$).

However, the magnesium ion-comprising material is preferably a magnesium carbonate-comprising material selected from the group consisting of dolomite $(CaMg(CO_3)_2)$, anhydrous magnesium carbonate or magnesite $(MgCO_3)$, hydromagnesite $(Mg_5(CO_3)_4(OH)_2 \cdot 4H_2O)$, artinite $(Mg_2(CO_3)(OH)_2 \cdot 3H_2O)$, dypingite $(Mg_5(CO_3)_4(OH)_2 \cdot 5H_2O)$, giorgiosite $(Mg_5(CO_3)_4(OH)_2 \cdot 5H_2O)$, pokrovskite $(Mg_2(CO_3)(OH)_2 \cdot 0.5H_2O)$, barringtonite $(MgCO_3 \cdot 2H_2O)$, lansfordite $(MgCO_3 \cdot 5H_2O)$, dolocarbonate and nesquehonite $(MgCO_3 \cdot 3H_2O)$.

In the meaning of the present invention, the term "dolocarbonate" refers to a composite material comprising a magnesium mineral, preferably hydromagnesite $(Mg_5(CO_3)_4(OH)_2 \cdot 4H_2O)$, and calcium carbonate agglomerated at primary particle level. Such dolocarbonates are for examples described in WO2013139957A1 and WO2015039994 A1, which are thus incorporated by references.

Preferably, the magnesium ion-comprising material encompasses a naturally occurring or synthetic magnesium ion-comprising material selected from the group consisting of periclase (MgO), anhydrous magnesium carbonate or magnesite $(MgCO_3)$, hydromagnesite $(Mg_5(CO_3)_4(OH)_2 \cdot 4H_2O)$, nesquehonite $(MgCO_3 \cdot 3H_2O)$, dolomite $(CaMg(CO_3)_2)$, dolocarbonate and mixtures thereof. For example, the magnesium ion-comprising material comprises the naturally occurring or synthetic magnesium carbonate selected from the group consisting of anhydrous magnesium carbonate or magnesite $(MgCO_3)$, hydromagnesite $(Mg_5(CO_3)_4(OH)_2 \cdot 4H_2O)$, nesquehonite $(MgCO_3 \cdot 3H_2O)$, dolomite $(CaMg(CO_3)_2)$, dolocarbonate and mixtures thereof in an amount of at least 80 wt.-%, more preferably at least 85 wt.-%, even more preferably between 85 and 100 wt.-%, and most preferably between 90 and 99.95 wt.-%, based on the total dry weight of the material.

For example, the magnesium ion-comprising material comprises periclase (MgO), anhydrous magnesium carbonate or magnesite $(MgCO_3)$ or dolomite $(CaMg(CO_3)_2)$ or hydromagnesite $(Mg_5(CO_3)_4(OH)_2 \cdot 4H_2O)$ or nesquehonite $(MgCO_3 \cdot 3H_2O)$, e.g. synthetic (or precipitated) hydromagnesite $(Mg_5(CO_3)_4(OH)_2 \cdot 4H_2O)$ or nesquehonite $(MgCO_3 \cdot 3H_2O)$, or naturally occurring anhydrous magnesium carbonate or magnesite $(MgCO_3)$ or dolomite $(CaMg(CO_3)_2)$. For example, the magnesium ion-comprising material comprises hydromagnesite $(Mg_5(CO_3)_4(OH)_2 \cdot 4H_2O)$, e.g. synthetic (or precipitated) hydromagnesite $(Mg_5(CO_3)_4(OH)_2 \cdot 4H_2O)$. In one embodiment, the magnesium ion-comprising material comprises anhydrous magnesium carbonate or magnesite $(MgCO_3)$ or dolomite $(CaMg(CO_3)_2)$ or hydromagnesite $(Mg_5(CO_3)_4(OH)_2 \cdot 4H_2O)$ or nesquehonite $(MgCO_3 \cdot 3H_2O)$, e.g. synthetic (or precipitated) hydromagnesite $(Mg_5(CO_3)_4(OH)_2 \cdot 4H_2O)$ or nesquehonite $(MgCO_3 \cdot 3H_2O)$ or naturally occurring anhydrous magnesium carbonate or magnesite $(MgCO_3)$ or dolomite $(CaMg(CO_3)_2)$, in an amount of at least 80 wt.-%, more preferably at least 85 wt.-%, even more preferably between 85 and 100 wt.-%, and most preferably between 90 and 99.95 wt.-%, based on the total dry weight of the material.

Preferably, the magnesium ion-comprising material comprises hydromagnesite $(Mg_5(CO_3)_4(OH)_2 \cdot 4H_2O)$, e.g. synthetic (or precipitated) hydromagnesite $(Mg_5(CO_3)_4(OH)_2 \cdot 4H_2O)$, in an amount of at least 80 wt.-%, more preferably at least 85 wt.-%, even more preferably between 85 and 100 wt.-%, and most preferably between 90 and 99.95 wt.-%, based on the total dry weight of the material.

In one embodiment, the magnesium ion-comprising material consists of anhydrous magnesium carbonate or magnesite $(MgCO_3)$ and/or dolomite $(CaMg(CO_3)_2)$, e.g. naturally occurring anhydrous magnesium carbonate or magnesite $(MgCO_3)$ or dolomite $(CaMg(CO_3)_2)$.

In an alternative embodiment, the magnesium ion-comprising material consists of hydromagnesite $(Mg_5(CO_3)_4(OH)_2 \cdot 4H_2O)$, e.g. synthetic (or precipitated) hydromagnesite $(Mg_5(CO_3)_4(OH)_2 \cdot 4H_2O)$.

In one embodiment, the magnesium ion-comprising material of step a) is hydromagnesite, e.g. natural or synthetic (or precipitated) hydromagnesite $(Mg_5(CO_3)_4(OH)_2 \cdot 4H_2O)$. Preferably, the magnesium ion-comprising material of step a) is synthetic (or precipitated) hydromagnesite $(Mg_5(CO_3)_4(OH)_2 \cdot 4H_2O)$.

It is appreciated that the magnesium ion-comprising material is preferably provided as particles not being in the nanosized range.

In general, the magnesium ion-comprising material has a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction.

Additionally or alternatively, the magnesium ion-comprising material has a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction.

Thus, the magnesium ion-comprising material has

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and

b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction.

For example, the magnesium ion-comprising material comprises periclase (MgO), anhydrous magnesium carbonate or magnesite ($MgCO_3$) or dolomite ($CaMg(CO_3)_2$) or hydromagnesite ($Mg_5(CO_3)_4(OH)_2 \cdot 4H_2O$), e.g. synthetic (or precipitated) hydromagnesite ($Mg_5(CO_3)_4(OH)_2 \cdot 4H_2O$) or naturally occurring anhydrous magnesium carbonate or magnesite ($MgCO_3$) or dolomite ($CaMg(CO_3)_2$), and has a volume median median particle ($d_{50}$) in the range from 1.9 to 10 $\mu$m, as determined by laser diffraction, and a volume top cut particle size ($d_{98}$) in the range from 10 to 40 $\mu$m, as determined by laser diffraction.

Preferably, the magnesium ion-comprising material comprises hydromagnesite ($Mg_5(CO_3)_4(OH)_2 \cdot 4H_2O$), e.g. synthetic (or precipitated) hydromagnesite ($Mg_5(CO_3)_4(OH)_2 \cdot 4H_2O$), and has a volume median particle size ($d_{50}$) in the range from 1.9 to 10 $\mu$m, as determined by laser diffraction, and a volume top cut particle size ($d_{98}$) in the range from 10 to 40 $\mu$m, as determined by laser diffraction.

In one embodiment, the magnesium ion-comprising material comprises periclase (MgO), anhydrous magnesium carbonate or magnesite ($MgCO_3$) or dolomite ($CaMg(CO_3)_2$) or hydromagnesite ($Mg_5(CO_3)_4(OH)_2 \cdot 4H_2O$), e.g. synthetic (or precipitated) hydromagnesite ($Mg_5(CO_3)_4(OH)_2 \cdot 4H_2O$) or naturally occurring anhydrous magnesium carbonate or magnesite ($MgCO_3$) or dolomite ($CaMg(CO_3)_2$), in an amount of at least 80 wt.-%, more preferably at least 85 wt.-%, even more preferably between 85 and 100 wt.-%, and most preferably between 90 and 99.95 wt.-%, based on the total dry weight of the material and has a volume median particle size ($d_{50}$) in the range from 1.9 to 10 $\mu$m, as determined by laser diffraction, and a volume top cut particle size ($d_{98}$) in the range from 10 to 40 $\mu$m, as determined by laser diffraction.

Preferably, the magnesium ion-comprising material comprises hydromagnesite ($Mg_5(CO_3)_4(OH)_2 \cdot 4H_2O$), e.g. synthetic (or precipitated) hydromagnesite ($Mg_5(CO_3)_4(OH)_2 \cdot 4H_2O$), in an amount of at least 80 wt.-%, more preferably at least 85 wt.-%, even more preferably between 85 and 100 wt.-%, and most preferably between 90 and 99.95 wt.-%, based on the total dry weight of the material and has a volume median particle size ($d_{50}$) in the range from 1.9 to 10 $\mu$m, as determined by laser diffraction, and a volume top cut particle size ($d_{98}$) in the range from 10 to 40 $\mu$m, as determined by laser diffraction.

Throughout the present document, the "particle size" of a magnesium ion-comprising material is described by its distribution of particle sizes on a volume base. Volume determined median grain diameter $d_{50}$ (or $d_{50}$(vol)) and the volume determined top cut particle size $d_{98}$ (or $d_{98}$(vol)) was evaluated using a Malvern Mastersizer 3000 Laser Diffraction System (Malvern Instruments Pic., Great Britain) equipped with a Hydro LV system. The $d_{50}$(vol) or $d_{98}$(vol) value indicates a diameter value such that 50 % or 98 % by volume, respectively, of the particles have a diameter of less than this value. The powders were suspended in 0.1 wt.-% $Na_4O_7P_2$ solution. 10 mL of 0.1 wt.-% $Na_4O_7P_2$ was added to the Hydro LV tank, then the sample slurry was introduced until an obscuration between 10-20 % was achieved. Measurements were conducted with red and blue light for 10 s each. For the analysis of the raw data, the models for non-spherical particle sizes using Mie theory was utilized, and a particle refractive index of 1.57, a density of 2.70 g/cm$^3$, and an absorption index of 0.005 was assumed. The methods and instruments are known to the skilled person and are commonly used to determine particle size distributions of fillers and pigments.

Additionally or alternatively, the magnesium ion-comprising has a BET specific surface area in the range from 10 to 100 m$^2$/g, preferably from 12 to 70 m$^2$/g, and most preferably from 17 to 60 m$^2$/g, measured using nitrogen and the BET method according to ISO 9277:2010.

The "specific surface area" (expressed in m$^2$/g) of a material as used throughout the present application can be determined by the Brunauer Emmett Teller (BET) method with nitrogen as adsorbing gas and by use of a ASAP 2460 instrument from Micromeritics. The method is well known to the skilled person and defined in ISO 9277:2010. Samples are conditioned at 150 °C under vacuum for a period of 60 min prior to measurement.

Additionally or alternatively, the magnesium ion-comprising material has an intra-particle intruded specific pore volume in the range from 0.9 to 2.3 cm$^3$/g, preferably from 1.2 to 2.1 cm$^3$/g, and most preferably from 1.5 to 2.0 cm$^3$/g, calculated from mercury porosimetry measurement.

The specific pore volume is measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 $\mu$m ($\sim$ nm). The equilibration time used at each pressure step is 20 seconds. The sample material is sealed in a 5 cm$^3$ chamber powder penetrometer for analysis. The data are corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

The total pore volume seen in the cumulative intrusion data can be separated into two regions with the intrusion data from 214 $\mu$m down to about 1 - 4 $\mu$m showing the coarse packing of the sample between any agglomerate structures contributing strongly. Below these diameters lies the fine interparticle packing of the particles themselves. If they also have intraparticle pores, then this region appears bi modal, and by taking the specific pore volume intruded by mercury into pores finer than the modal turning point, i.e. finer than the bi-modal point of inflection, we thus define the specific intraparticle pore volume. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired pore volume of the internal pores alone in terms of the pore volume per unit mass (specific pore volume). The same principle of subtraction, of course, applies for isolating any of the other pore size regions of interest.

Thus, the magnesium ion-comprising material has

a) a BET specific surface area in the range from 10 to 100 $m^2/g$, preferably from 12 to 70 $m^2/g$, and most preferably from 17 to 60 $m^2/g$, measured using nitrogen and the BET method according to ISO 9277:2010, and
b) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

In one embodiment, the magnesium ion-comprising material thus has

a) a volume median particle size $d_{50}$ in the range from 1 to 75 $\mu m$, preferably from 1.2 to 50 $\mu m$, more preferably from 1.5 to 30 $\mu m$, even more preferably from 1.7 to 15 $\mu m$ and most preferably from 1.9 to 10 $\mu m$, as determined by laser diffraction, and/or
b) a volume top cut particle size $d_{98}$ in the range from 2 to 150 $\mu m$, preferably from 4 to 100 $\mu m$, more preferably from 6 to 80 $\mu m$, even more preferably from 8 to 60 $\mu m$ and most preferably from 10 to 40 $\mu m$, as determined by laser diffraction; and/or
c) a BET specific surface area in the range from 10 to 100 $m^2/g$, preferably from 12 to 70 $m^2/g$, and most preferably from 17 to 60 $m^2/g$, measured using nitrogen and the BET method according to ISO 9277:2010; and/or
d) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

For example, the magnesium ion-comprising material is hydromagnesite ($Mg_5(CO_3)_4(OH)_2 \cdot 4H_2O$), e.g. synthetic (or precipitated) hydromagnesite ($Mg_5(CO_3)_4(OH)_2 \cdot 4H_2O$), having

a) a volume median particle size $d_{50}$ in the range from 1 to 75 $\mu m$, preferably from 1.2 to 50 $\mu m$, more preferably from 1.5 to 30 $\mu m$, even more preferably from 1.7 to 15 $\mu m$ and most preferably from 1.9 to 10 $\mu m$, as determined by laser diffraction, and/or
b) a volume top cut particle size $d_{98}$ in the range from 2 to 150 $\mu m$, preferably from 4 to 100 $\mu m$, more preferably from 6 to 80 $\mu m$, even more preferably from 8 to 60 $\mu m$ and most preferably from 10 to 40 $\mu m$, as determined by laser diffraction; and/or
c) a BET specific surface area in the range from 10 to 100 $m^2/g$, preferably from 12 to 70 $m^2/g$, and most preferably from 17 to 60 $m^2/g$, measured using nitrogen and the BET method according to ISO 9277:2010; and/or
d) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

Preferably, the magnesium ion-comprising material has

a) a volume median particle size $d_{50}$ in the range from 1 to 75 $\mu m$, preferably from 1.2 to 50 $\mu m$, more preferably from 1.5 to 30 $\mu m$, even more preferably from 1.7 to 15 $\mu m$ and most preferably from 1.9 to 10 $\mu m$, as determined by laser diffraction, and
b) a volume top cut particle size $d_{98}$ in the range from 2 to 150 $\mu m$, preferably from 4 to 100 $\mu m$, more preferably from 6 to 80 $\mu m$, even more preferably from 8 to 60 $\mu m$ and most preferably from 10 to 40 $\mu m$, as determined by laser diffraction; and
c) a BET specific surface area in the range from 10 to 100 $m^2/g$, preferably from 12 to 70 $m^2/g$, and most preferably from 17 to 60 $m^2/g$, measured using nitrogen and the BET method according to ISO 9277:2010; and
d) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

For example, the magnesium ion-comprising material is hydromagnesite ($Mg_5(CO_3)_4(OH)_2 \cdot 4H_2O$), e.g. synthetic (or precipitated) hydromagnesite ($Mg_5(CO_3)_4(OH)_2 \cdot 4H_2O$), having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and

b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and

c) a BET specific surface area in the range from 10 to 100 $m^2$/g, preferably from 12 to 70 $m^2$/g, and most preferably from 17 to 60 $m^2$/g, measured using nitrogen and the BET method according to ISO 9277:2010; and

d) an intra-particle intruded specific pore volume in the range from 0.9 to 2.3 $cm^3$/g, preferably from 1.2 to 2.1 $cm^3$/g, and most preferably from 1.5 to 2.0 $cm^3$/g, calculated from mercury porosimetry measurement.

It is appreciated that the magnesium ion-comprising material is preferably a magnesium carbonate-comprising material.

The magnesium carbonate-comprising material can be a naturally occurring or synthetic magnesium carbonate-comprising material.

For example, the magnesium carbonate-comprising material encompasses naturally occurring or synthetic magnesium carbonate selected from the group comprising magnesite ($MgCO_3$), hydromagnesite ($Mg_5(CO_3)_4(OH)_2 \cdot 4H_2O$), artinite ($Mg_2(CO_3)(OH)_2 \cdot 3H_2O$), dypingite ($Mg_5(CO_3)_4(OH)_2 \cdot 5H_2O$), giorgiosite ($Mg_5(CO_3)_4(OH)_2 \cdot 5H_2O$), pokrovskite ($Mg_2(CO_3)(OH)_2 \cdot 0.5H_2O$), barringtonite ($MgCO_3 \cdot 2H_2O$), lansfordite ($MgCO_3 \cdot 5H_2O$), nesquehonite ($MgCO_3 \cdot 3H_2O$), dolocarbonate and mixtures thereof.

Preferably, the magnesium carbonate-comprising material encompasses a synthetic magnesium carbonate-comprising material selected from the group comprising magnesite ($MgCO_3$), hydromagnesite ($Mg_5(CO_3)_4(OH)_2 \cdot 4H_2O$), artinite ($Mg_2(CO_3)(OH)_2 \cdot 3H_2O$), dypingite ($Mg_5(CO_3)_4(OH)_2 \cdot 5H_2O$), giorgiosite ($Mg_5(CO_3)_4(OH)_2 \cdot 5H_2O$), pokrovskite ($Mg_2(CO_3)(OH)_2 \cdot 0.5H_2O$), barringtonite ($MgCO_3 \cdot 2H_2O$), lansfordite ($MgCO_3 \cdot 5H_2O$), nesquehonite ($MgCO_3 \cdot 3H_2O$), dolocarbonate and mixtures thereof.

In one embodiment, the magnesium carbonate-comprising material comprises synthetic hydromagnesite ($Mg_5(CO_3)_4(OH)_2 \cdot 4H_2O$). Preferably, the magnesium carbonate-comprising material comprises synthetic hydromagnesite ($Mg_5(CO_3)_4(OH)_2 \cdot 4H_2O$) in an amount of at least 80 wt.-%, more preferably at least 85 wt.-%, even more preferably between 85 and 100 wt.-%, and most preferably between 90 and 99.95 wt.-%, based on the total dry weight of the material.

It is to be noted that synthetic hydromagnesite may be also called precipitated hydromagnesite.

The magnesium carbonate-comprising material is in the form of a particulate material, and may have a particle size distribution as conventionally employed for the material(s) involved in the type of product to be produced. In general, it is preferred that the magnesium carbonate-comprising material has a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction.

Additionally or alternatively, the magnesium carbonate-comprising material has a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, as determined by laser diffraction.

Thus, the magnesium carbonate-comprising material preferably has a

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and

b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, as determined by laser diffraction.

In one embodiment, the magnesium carbonate-comprising material has a volume median particle size $d_{50}$ in the range from 1.9 to 10 $\mu$m, as determined by laser diffraction, and a volume top cut particle size $d_{98}$ in the range from 10 to 40 $\mu$m, as determined by laser diffraction.

For example, the magnesium carbonate-comprising material is synthetic (or precipitated) hydromagnesite ($Mg_5(CO_3)_4(OH)_2 \cdot 4H_2O$) and has a volume median particle size $d_{50}$ in the range from 1.9 to 10 $\mu$m, as determined by laser diffraction, and a volume top cut particle size $d_{98}$ in the range from 10 to 40 $\mu$m, as determined by laser diffraction.

Additionally or alternatively, the magnesium carbonate-comprising material has a BET specific surface area in the range from 10 to 100 $m^2$/g, preferably from 12 to 70 $m^2$/g, and most preferably from 17 to 60 $m^2$/g, measured using nitrogen and the BET method according to ISO 9277:2010.

Additionally or alternatively, it is specifically advantageous if the magnesium carbonate-comprising material has a high intra-particle intruded specific pore volume. For example, it is preferred that the magnesium carbonate-comprising material has an intra-particle intruded specific pore volume in the range from 0.9 to 2.3 $cm^3/g$, calculated from mercury intrusion porosimetry. In one embodiment, the magnesium carbonate-comprising material has an intra-particle intruded specific pore volume in the range from 1.2 to 2.1 $cm^3/g$, and most preferably from 1.5 to 2.0 $cm^3/g$, calculated from mercury intrusion porosimetry.

Preferably, the magnesium carbonate-comprising material has a specific surface area in the range from 17 to 60 $m^2/g$, measured using nitrogen and the BET method according to ISO 9277:2010, and an intra-particle intruded specific pore volume in the range from 1.5 to 2.0 $cm^3/g$, calculated from mercury porosimetry measurement.

According to one embodiment, the magnesium carbonate-comprising material has

> a) a specific surface area in the range from 10 to 100 $m^2/g$, more preferably from 12 to 70 $m^2/g$, and most preferably from 17 to 60 $m^2/g$, measured using nitrogen and the BET method according to ISO 9277:2010, and
> b) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

Preferably, the magnesium carbonate-comprising material has

> a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and
> b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and
> c) a BET specific surface area in the range from 10 to 100 $m^2/g$, preferably from 12 to 70 $m^2/g$, and most preferably from 17 to 60 $m^2/g$, measured using nitrogen and the BET method according to ISO 9277:2010; and
> d) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

In one embodiment, the magnesium ion-comprising material of step a) is a surface-reacted magnesium carbonate-comprising material.

In particular, the surface-reacted magnesium carbonate-comprising material is obtained by treating the surface of the magnesium carbonate-comprising material with one or more compound(s) selected from the group consisting of sulphuric acid, phosphoric acid, carbonic acid, carboxylic acids containing up to six carbon atoms, preferably selected from formic acid, acetic acid, propionic acid, lactic acid and mixtures thereof; and di-, and tri-carboxylic acids where the carboxylic acid groups are linked by a chain of 0-4 intermittent carbon atoms, preferably selected from oxalic acid, citric acid, succinic acid, maleic acid, malonic acid, tartaric acid, adipic acid, fumaric acid and mixtures thereof, or a corresponding salt thereof.

Accordingly, it should be noted that the surface-reacted magnesium carbonate-comprising material is obtained by treating the surface of the magnesium carbonate-comprising material with one compound. Alternatively, the surface-reacted magnesium carbonate-comprising material is obtained by treating the surface of the magnesium carbonate-comprising material with two or more compounds. For example, the surface-reacted magnesium carbonate-comprising material is obtained by treating the surface of the magnesium carbonate-comprising material with two or three or four compounds, like two compounds.

In one embodiment of the present invention, the surface-reacted magnesium carbonate-comprising material is obtained by treating the surface of the magnesium carbonate-comprising material with two compounds.

According to one embodiment, the surface-reacted magnesium carbonate-comprising material is obtained by treating the surface of the magnesium carbonate-comprising material with sulphuric acid.

In one embodiment, the surface-reacted magnesium carbonate-comprising material is obtained by treating the surface of the magnesium carbonate-comprising material with a salt of sulphuric acid, e.g. an alkali metal salt of sulphuric acid. For example, the alkali metal salt of sulphuric acid is sodium sulphate or potassium sulphate, preferably sodium sulphate.

Additionally or alternatively, the surface-reacted magnesium carbonate-comprising material is obtained by treating the surface of the magnesium carbonate-comprising material with phosphoric acid.

In one embodiment, the surface-reacted magnesium carbonate-comprising material is obtained by treating the surface of the magnesium carbonate-comprising material with a salt of phosphoric acid, e.g. an alkali metal salt of phosphoric acid. For example, the alkali metal salt of phosphoric acid is sodium phosphate or potassium phosphate, preferably sodium phosphate.

Additionally or alternatively, the surface-reacted magnesium carbonate-comprising material is obtained by treating the surface of the magnesium carbonate-comprising material with carbonic acid.

In one embodiment, the surface-reacted magnesium carbonate-comprising material is obtained by treating the surface of the magnesium carbonate-comprising material with a salt of carbonic acid, e.g. an alkali metal salt of carbonic acid. For example, the alkali metal salt of carbonic acid is sodium carbonate or potassium carbonate, preferably sodium carbonate.

Additionally or alternatively, the surface-reacted magnesium carbonate-comprising material is obtained by treating the surface of the magnesium carbonate-comprising material with a carboxylic acid containing up to six carbon atoms.

The carboxylic acid containing up to six carbon atoms is preferably an aliphatic carboxylic acid and may be selected from one or more linear chain, branched chain, saturated, unsaturated and/or alicyclic carboxylic acids. Preferably, the carboxylic acid containing up to six carbon atoms is a monocarboxylic acid, i.e. the carboxylic acid containing up to six carbon atoms is characterized in that a single carboxyl group is present. Said carboxyl group is preferably placed at the end of the carbon skeleton.

In one embodiment of the present invention, the carboxylic acid containing up to six carbon atoms is preferably selected from the group consisting of formic acid, acetic acid, propionic acid, lactic acid, butanoic acid, pentanoic acid, hexanoic acid and mixtures thereof. More preferably, the carboxylic acid containing up to six carbon atoms is selected from the group consisting of formic acid, acetic acid, propionic acid, lactic acid, butanoic acid and mixtures thereof.

For example, the carboxylic acid containing up to six carbon atoms is selected from the group consisting of formic acid, acetic acid, propionic acid, lactic acid, and mixtures thereof. Preferably, the carboxylic acid containing up to six carbon atoms is selected from the group consisting of acetic acid, propionic acid and mixtures thereof.

In one embodiment, the carboxylic acid containing up to six carbon atoms is acetic acid.

In one embodiment, the surface-reacted magnesium carbonate-comprising material is obtained by treating the surface of the magnesium carbonate-comprising material with a salt of the carboxylic acid containing up to six carbon atoms, e.g. an alkali metal salt of the carboxylic acid containing up to six carbon atoms. For example, the alkali metal salt of the carboxylic acid containing up to six carbon atoms is a sodium or potassium salt, preferably sodium salt, of formic acid, acetic acid, propionic acid, lactic acid, and mixtures thereof.

Additionally or alternatively, the surface-reacted magnesium carbonate-comprising material is obtained by treating the surface of the magnesium carbonate-comprising material with a di-, and/or tri-carboxylic acid containing up to six carbon atoms where the carboxylic acid groups are linked by a chain of 0-4 intermittent carbon atoms.

The dicarboxylic acid containing up to six carbon atoms is characterized in that two carboxyl groups are present. Said carboxyl groups are preferably placed at each end of the carbon skeleton with the proviso that the carboxylic acid groups are linked by a chain of 0-4 intermittent carbon atoms.

In one embodiment of the present invention, the dicarboxylic acid containing up to six carbon atoms is preferably selected from the group consisting of oxalic acid, malonic acid, maleic acid, succinic acid, tartaric acid, glutaric acid, adipic acid, fumaric acid, and mixtures thereof. More preferably, the dicarboxylic acid containing up to six carbon atoms is selected from the group consisting of oxalic acid, succinic acid, maleic acid, malonic acid, tartaric acid, adipic acid, fumaric acid and mixtures thereof.

In one embodiment, the surface-reacted magnesium carbonate-comprising material is obtained by treating the surface of the magnesium carbonate-comprising material with a salt of the dicarboxylic acid containing up to six carbon atoms, e.g. an alkali metal salt of the dicarboxylic acid containing up to six carbon atoms. For example, the alkali metal salt of the dicarboxylic acid containing up to six carbon atoms is a sodium salt of oxalic acid, succinic acid, maleic acid, malonic acid, tartaric acid, adipic acid and/or fumaric acid, preferably a sodium salt of oxalic acid, maleic acid, malonic acid and/or fumaric acid. It is appreciated that the salt of the dicarboxylic acid containing up to six carbon atoms can be a monobasic or dibasic salt of the dicarboxylic acid.

The tricarboxylic acid containing up to six carbon atoms is characterized in that three carboxyl groups are present. Two carboxyl groups are placed at each end of the carbon skeleton with the proviso that the two carboxylic acid groups are linked by a chain of 0-4 intermittent carbon atoms.

In one embodiment of the present invention, the tricarboxylic acid containing up to six carbon atoms is preferably selected from the group consisting of citric acid, isocitric acid, aconitic acid and mixtures thereof. More preferably, the tricarboxylic acid containing up to six carbon atoms is selected from citric acid and/or isocitric acid.

Most preferably, the tricarboxylic acid containing up to six carbon atoms is citric acid.

In one embodiment, the surface-reacted magnesium carbonate-comprising material is obtained by treating the surface of the magnesium carbonate-comprising material with a salt of the tricarboxylic acid containing up to six carbon atoms, e.g. an alkali metal salt of the tricarboxylic acid containing up to six carbon atoms. For example, the alkali metal salt of the tricarboxylic acid containing up to six carbon atoms is sodium citrate or potassium citrate, preferably sodium citrate. It is appreciated that the salt of the tricarboxylic acid containing up to six carbon atoms can be a monobasic or dibasic or tribasic salt of the tricarboxylic acid. For example, the salt of the tricarboxylic acid containing up to six carbon atoms can be a monobasic or dibasic or tribasic citrate, such as monobasic or dibasic or tribasic sodium citrate.

In one embodiment, the surface-reacted magnesium carbonate-comprising material is obtained by treating the surface of the magnesium carbonate-comprising material with one or more compound(s) being di-, and tri-carboxylic acids where the carboxylic acid groups are linked by a chain of 0-4 intermittent carbon atoms, preferably selected from oxalic acid, citric acid, succinic acid, maleic acid, malonic acid, tartaric acid, adipic acid, fumaric acid and mixtures thereof. Alternatively, the surface-reacted magnesium carbonate-comprising material is obtained by treating the surface of the magnesium carbonate-comprising material with one or more compound(s) being a sodium salt of di- and tri-carboxylic acids where the carboxylic acid groups are linked by a chain of 0-4 intermittent carbon atoms, preferably selected from oxalic acid, citric acid, succinic acid, maleic acid, malonic acid, tartaric acid, adipic acid, fumaric acid and mixtures thereof.

Preferably, the surface-reacted magnesium carbonate-comprising material is obtained by treating the surface of the magnesium carbonate-comprising material with phosphoric acid or an alkali metal salt of phosphoric acid, such as sodium phosphate, more preferably an alkali metal salt of phosphoric acid, such as sodium phosphate. Alternatively, the surface-reacted magnesium carbonate-comprising material is obtained by treating the surface of the magnesium carbonate-comprising material with sulphuric acid or an alkali metal salt of sulphuric acid, such as sodium sulphate, more preferably sodium sulphate. Alternatively, the surface-reacted magnesium carbonate-comprising material is obtained by treating the surface of the magnesium carbonate-comprising material with citric acid or an alkali metal salt of citric acid, such as sodium citrate, more preferably an alkali metal salt of citric acid, such as sodium citrate.

In view of the above, the surface of the magnesium carbonate-comprising material preferably comprises one or more compound(s) selected from the group consisting of sulphuric acid, phosphoric acid, carbonic acid, carboxylic acids containing up to six carbon atoms, preferably selected from formic acid, acetic acid, propionic acid, lactic acid and mixtures thereof; and di-, and tri-carboxylic acids where the carboxylic acid groups are linked by a chain of 0-4 intermittent carbon atoms, preferably selected from oxalic acid, citric acid, succinic acid, maleic acid, malonic acid, tartaric acid, adipic acid, fumaric acid and mixtures thereof, or a corresponding salt thereof and/or reaction products thereof.

The term "reaction products" in the meaning of the present invention refers to products obtained by contacting the surface of the magnesium carbonate-comprising material with one or more compound(s) selected from the group consisting of sulphuric acid, phosphoric acid, carbonic acid, carboxylic acids containing up to six carbon atoms, preferably selected from formic acid, acetic acid, propionic acid, lactic acid and mixtures thereof; and di-, and tri-carboxylic acids where the carboxylic acid groups are linked by a chain of 0-4 intermittent carbon atoms, preferably selected from oxalic acid, citric acid, succinic acid, maleic acid, malonic acid, tartaric acid, adipic acid, fumaric acid and mixtures thereof, or a corresponding salt thereof. Said reaction products are formed between the applied one or more compound(s) and reactive molecules located at the surface of the magnesium carbonate-comprising material.

It is appreciated that the surface-reacted magnesium carbonate-comprising material is preferably obtained by treating the surface of the magnesium carbonate-comprising material with the one or more compound(s) or a corresponding salt thereof in an amount from 0.1 to 20 wt.-%, based on the total dry weight of the magnesium carbonate-comprising material. For example, the surface-reacted magnesium carbonate-comprising material is preferably obtained by treating the surface of the magnesium carbonate-comprising material with the one or more compound(s) or a corresponding salt thereof in an amount from 0.1 to 15 wt.-%, based on the total dry weight of the magnesium carbonate-comprising material. Preferably, the surface-reacted magnesium carbonate-comprising material is preferably obtained by treating the surface of the magnesium carbonate-comprising material with the one or more compound(s) or a corresponding salt thereof in an amount from 0.5 to 15 wt.-%, based on the total dry weight of the magnesium carbonate-comprising material.

In general, the surface-reacted magnesium carbonate-comprising material can be prepared by any known method suitable for obtaining a treatment layer of one or more compound(s) on the surface of filler materials such as magnesium carbonate-comprising material.

For example, the surface-reacted magnesium carbonate-comprising material is prepared by a method comprising at least the steps of:

i) providing a magnesium carbonate-comprising material,
ii) providing one or more compound(s) selected from the group consisting of sulphuric acid, phosphoric acid, carbonic acid, carboxylic acids containing up to six carbon atoms, preferably selected from formic acid, acetic acid, propionic acid, lactic acid and mixtures thereof; and di-, and tri-carboxylic acids where the carboxylic acid groups are linked by a chain of 0-4 intermittent carbon atoms, preferably selected from oxalic acid, citric acid, succinic acid, maleic acid, malonic acid, tartaric acid, adipic acid, fumaric acid and mixtures thereof, or a corresponding salt thereof, and
iii) treating the surface of the magnesium carbonate-comprising material of step a), under mixing, in one or more steps, with the one or more compound(s) or a corresponding salt thereof of step b) such that a reaction is achieved by the one or more compound(s) or the corresponding salt thereof and the surface of said magnesium carbonate-comprising material.

For example, the surface-reacted magnesium carbonate-comprising material is prepared in a dry method, e.g. by applying the one or more compound(s) onto the surface of the magnesium carbonate-comprising material without using solvents. If the one or more compound(s) are in a solid state, the one or more compound(s) may be heated in order to provide them in a liquid state for ensuring an essentially even distribution of the one or more compound(s) on the surface of the magnesium carbonate-comprising material.

Alternatively, the surface-reacted magnesium carbonate-comprising material is prepared in a wet method, e.g. by dissolving the one or more compound(s) in a solvent and applying the mixture onto the surface of the magnesium carbonate-comprising material. Optionally the mixture comprising the solvent and the one or more compound(s) may be heated. If the one or more compound(s) are dissolved in a solvent, the solvent is preferably an organic solvent, preferably selected from toluene, acetone and ethanol.

In general, the step of treating the surface of the magnesium carbonate-comprising material with the one or more compound(s) or a corresponding salt thereof may be carried out by any method suitable for achieving an essentially even distribution of the one or more compound(s) and thus a reaction on the surface of the magnesium carbonate-comprising material. Thus, the one or more compound(s) and the magnesium carbonate-comprising material are mixed, preferably agitated or shaken, to facilitate and accelerate the preparation of the surface-reacted magnesium carbonate-comprising material, e.g. by using a mixing device, spray coater or encapsulation processes. If a solvent is used, the obtained surface-reacted magnesium carbonate-comprising material may be dried to remove the volatile components, preferably under vacuum.

In the dry and wet method, the step of treating the surface of the magnesium carbonate-comprising material with the one or more compound(s) or a corresponding salt thereof such that a reaction is achieved by the one or more compound(s) or the corresponding salt thereof and the surface of said magnesium carbonate-comprising material may be carried out in a single step or in at least two steps.

According to one embodiment of the present invention, the surface-reacted magnesium carbonate-comprising material is thus prepared by means of one or more of the following methods:

(i) dry treatment, i.e. treating the surface of the magnesium carbonate-comprising material with the one or more compound(s) which is/are in neat form, preferably in a mixing device or by using a spray coater;
(ii) wet treatment, i.e. treating the surface of the magnesium carbonate-comprising material with the one or more compound(s) which is/are dissolved in a solvent, optionally under heating, preferably in a mixing device or by using a spray coater; or
(iii) melt dry treatment, i.e. treating the surface of the magnesium carbonate-comprising material with a melt of the one or more compound(s) which is/are in neat form in a heated mixer (e.g. a fluid bed mixer).

The surface-reacted magnesium carbonate-comprising material obtained has preferably a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction. According to a further embodiment of the present invention, the surface-reacted magnesium carbonate-comprising material is in form of particles having a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, as determined by laser diffraction.

Thus, the surface-reacted magnesium carbonate-comprising material has a

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and
b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, as determined by laser diffraction.

In one embodiment, the surface-reacted magnesium carbonate-comprising material has a volume median particle size $d_{50}$ in the range from 1.9 to 10 $\mu$m, as determined by laser diffraction, and a volume top cut particle size $d_{98}$ in the range from 10 to 40 $\mu$m, as determined by laser diffraction.

For example, the surface-reacted magnesium carbonate-comprising material is obtained by treating the surface of synthetic (or precipitated) hydromagnesite ($Mg_5(CO_3)_4(OH)_2 \cdot 4H_2O$) with one or more compound(s) selected from the group consisting of sulphuric acid, phosphoric acid, carbonic acid, carboxylic acids containing up to six carbon atoms, preferably selected from formic acid, acetic acid, propionic acid, lactic acid and mixtures thereof; and di-, and tri-carboxylic acids where the carboxylic acid groups are linked by a chain of 0-4 intermittent carbon atoms, preferably selected from oxalic acid, citric acid, succinic acid, maleic acid, malonic acid, tartaric acid, adipic acid, fumaric acid and mixtures thereof, or a corresponding salt thereof and has a volume median particle size $d_{50}$ in the range from 1.9 to 10 $\mu$m, as determined by laser diffraction, and a volume top cut particle size $d_{98}$ in the range from 10 to 40 $\mu$m, as determined by laser diffraction.

In one embodiment, the surface-reacted magnesium carbonate-comprising material has a BET specific surface area in the range from 10 to 100 $m^2$/g, preferably from 12 to 70 $m^2$/g, and most preferably from 17 to 60 $m^2$/g, measured using nitrogen and the BET method according to ISO 9277:2010.

Additionally or alternatively, the surface-reacted magnesium carbonate-comprising material has an intra-particle intruded specific pore volume in the range from 0.9 to 2.3 $cm^3$/g, preferably from 1.2 to 2.1 $cm^3$/g, and most preferably from 1.5 to 2.0 $cm^3$/g, calculated from mercury porosimetry measurement.

For example, the surface-reacted magnesium carbonate-comprising material has

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and

b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and

c) a BET specific surface area in the range from 10 to 100 $m^2$/g, preferably from 12 to 70 $m^2$/g, and most preferably from 17 to 60 $m^2$/g, measured using nitrogen and the BET method according to ISO 9277:2010; and

d) an intra-particle intruded specific pore volume in the range from 0.9 to 2.3 $cm^3$/g, preferably from 1.2 to 2.1 $cm^3$/g, and most preferably from 1.5 to 2.0 $cm^3$/g, calculated from mercury porosimetry measurement.

Preferably, the surface-reacted magnesium carbonate-comprising material is surface-reacted synthetic (or precipitated) hydromagnesite ($Mg_5(CO_3)_4(OH)_2 \cdot 4H_2O$) having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and

b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and

c) a BET specific surface area in the range from 10 to 100 $m^2$/g, preferably from 12 to 70 $m^2$/g, and most preferably from 17 to 60 $m^2$/g, measured using nitrogen and the BET method according to ISO 9277:2010; and

d) an intra-particle intruded specific pore volume in the range from 0.9 to 2.3 $cm^3$/g, preferably from 1.2 to 2.1 $cm^3$/g, and most preferably from 1.5 to 2.0 $cm^3$/g, calculated from mercury porosimetry measurement.

As stated above, the magnesium ion-comprising material of step a) is provided in form of an aqueous suspension.

Preferably, the aqueous suspension of step a) has a solids content in the range from 1 to 40 wt.-%, preferably from 5 to 35 wt.-%, and most preferably from 7 to 26 wt.-%, based on the total weight of the aqueous suspension.

For the purpose of the present invention, a "suspension" or "slurry" refers to a system comprising a liquid, i.e. an aqueous solvent, and particles of the magnesium ion-comprising material, wherein the particles of the magnesium ion-comprising material are present as solids in the liquid. The aqueous suspension is more viscous and can be of higher density than the liquid from which it is formed.

The "liquid" is typically an "aqueous solvent" which does not exclude that the aqueous solvent comprises minor amounts of at least one water-miscible solvent. For example, the at least one water-miscible solvent is preferably selected from methanol, ethanol, acetone, acetonitrile, tetrahydrofuran and mixtures thereof. In one embodiment of the present invention, the liquid comprises water in an amount of at least 80 wt.-%, preferably at least 90 wt.-%, more preferably at least 95 wt.-%, even more preferably at least 99 wt.-%, based on the total weight of the aqueous solvent. Preferably, the aqueous solvent consists of water, i.e. the amount of water is 100 wt.-%, based on the total weight of the liquid.

It is further preferred that the aqueous suspension provided in step a) has a Brookfield viscosity at 100 rpm from 25 to 1 000 mPas at a temperature of +23°C ($\pm$ 2°C), preferably from 25 to 700 mPas at +23°C ($\pm$ 2°C), more preferably from 25 to 500 mPas at +23°C ($\pm$ 2°C) and most preferably from 50 to 300 mPas at +23°C ($\pm$ 2°C).

According to step b) of the present method, the aqueous suspension comprising a magnesium ion-comprising material of step a) is homogenized.

"Homogenizing" in the meaning of the present invention refers to a step of making the particles of the magnesium ion-comprising material in the aqueous suspension of step a) leads to stronger aggregates after drying in terms of granule stability.

The homogenizing can be carried out by using various methods which are well known in the art.

The homogenizing equipment may be selected from the conventionally used ones for homogenizing purposes. Thus, the homogenizing device may be selected from the group comprising a piston pump, a high-shear apparatus and the like. For example, a GEA Ariete NS3055 of GEA Mechanical Equipment Italia S.p.A. can be used for homogenizing in step b).

Alternatively, the homogenizing in step b) is carried out by milling. The homogenizing in step b) can be carried out in milling or kneading devices well known in the art. Thus, the milling or kneading device may be selected from the horizontal and vertical mills conventionally used for milling purposes or kneaders conventionally used for kneading purposes. For example, the milling device may be selected from a horizontal and/or a vertical stirred media mill, preferably a vertical stirred media mill, a horizontal and/or a vertical agitated bead mill such as a Dyno-KDL bead mill, a Netzsch LabStar or LMZ-type mill or a LME-type mill; a sand mill and the like. For example, the kneading device may be selected from a Sigma-Kneader, planetary mixer and the like.

It might be noted that there may be differences as regards the particle size distributions to be achieved depending on the method used.

The homogenizing in step b) is carried out once or several times. It is appreciated that the number of times for carrying out step b) depends mainly on the pressure used and the magnesium ion-comprising material obtained in step b). The skilled person can thus easily adapt the number of times for carrying out step b) in accordance with the equipment or conditions used during step b). Thus, the homogenizing in step b) can be carried out in recirculation mode.

It is preferred that the homogenizing in step b) is carried out 1 to 5 times, more preferably 1 to 3 times, i.e. once, twice or three times, even more preferably once or twice. Most preferably, the homogenizing in step b) is carried out twice.

It is appreciated that the homogenizing in step b) is preferably carried out by using a highpressure homogenizer.

In one embodiment, the homogenizing in step b) is carried out at a pressure ranging from 50 to 900 bar, preferably from 100 to 750 bar, and most preferably from 130 to 650 bar.

Additionally or alternatively, the homogenizing in step b) is carried out at an initial temperature ranging from 5 to 95°C, preferably from 10 to 80°C, and most preferably from 15 to 60°C.

Thus, it is preferred that the homogenizing in step b) is carried out at

  a) a pressure ranging from 50 to 900 bar, preferably from 100 to 750 bar, and most preferably from 130 to 650 bar, or
  b) an initial temperature ranging from 5 to 95°C, preferably from 10 to 80°C, and most preferably from 15 to 60°C.

More preferably, the homogenizing in step b) is carried out at

  a) a pressure ranging from 50 to 900 bar, preferably from 100 to 750 bar, and most preferably from 130 to 650 bar, and
  b) an initial temperature ranging from 5 to 95°C, preferably from 10 to 80°C, and most preferably from 15 to 60°C.

Preferably, the aqueous suspension obtained in step b) has a solids content in the range from 1 to 40 wt.-%, preferably from 5 to 35 wt.-%, and most preferably from 7 to 26 wt.-%, e.g. from 10 to 25 wt.-% or from 15 to 25 wt.-%, based on the total weight of the aqueous suspension, in step b).

It is appreciated that the homogenizing in step b) may result in an increase of the solids content in the aqueous suspension compared to the aqueous suspension subjected to step b). For example, the aqueous suspension obtained in homogenizing step b) may have a solids content being at least 1 %, more preferably at least 2 % and most preferably at least 3 %, e.g. from 3 to 4 % above the solids content of the aqueous suspension subjected to step b). This is especially applicable if step b) is carried out in a homogenizer.

If step b) is carried out by milling, the aqueous suspension obtained in homogenizing step b) preferably has a solids content being at most 3 %, more preferably at most 2 % and most preferably at most 1 %, above the solids content of the aqueous suspension subjected to step b).

It is appreciated that the homogenizing by milling is preferably carried out at a specific energy ranging from 10 to 125 kWh/ton of dry product, preferably from 25 to 100 kWh/ton of dry product.

Additionally or alternatively, the homogenizing by milling is carried out an initial temperature ranging from 5 to 95°C, preferably from 10 to 80°C, and most preferably from 15 to 60°C.

Thus, it is preferred that the homogenizing by milling in step b) is carried out at

  a) a specific energy ranging from 10 to 125 kWh/ton of dry product, preferably from 25 to 100 kWh/ton of dry product, or
  b) an initial temperature ranging from 5 to 95°C, preferably from 10 to 80°C, and most preferably from 15 to 60°C.

More preferably, the homogenizing by milling in step b) is carried out at

  a) a specific energy ranging from 10 to 125 kWh/ton of dry product, preferably from 25 to 100 kWh/ton of dry product, and
b) an initial temperature ranging from 5 to 95°C, preferably from 10 to 80°C, and most preferably from 15 to 60°C.

In one embodiment, at least one disintegrant is added before and/or during and/or after step b). Preferably, the at least one disintegrant is added before or during or after step b), more preferably before or after step b). Most preferably, the at least one disintegrant is added after step b).

In one embodiment of the present invention, the at least one disintegrant comprises, preferably consists of, one disintegrant. Alternatively, the at least one disintegrant comprises, preferably consists of, two or more disintegrants. For example, the at least one disintegrant comprises, preferably consists of, two or three disintegrants.

Preferably, the at least one disintegrant comprises, preferably consists of, one disintegrant.

It is to be noted that the disintegrant(s) which may be used in the method of the present invention generally are those well-known in the art of granulation.

It is to be noted that any compound known as disintegrant or which may act as a disintegrant may be used in the method of the present invention.

In a preferred embodiment, the at least one disintegrant may be selected from the group comprising sodium croscarmellose, modified cellulose gums, insoluble cross-linked polyvinylpyrrolidones, starches, modified starches, starch glycolates such as sodium starch glycolate, micro crystalline cellulose, pregelatinized starch, sodium carboxymethyl starch, low-substituted hydroxypropyl cellulose, homopolymers of N-vinyl-2-pyrrolidone, alkyl-, hydroxyalkyl-, carboxyalkyl-cellulose esters, alginic acid, microcrystalline cellulose and its polymorphic forms, ion exchange resins, gums, chitin, chitosan, clays, gellan gum, crosslinked polacrillin copolymers, agar, gelatine, dextrines, acrylic acid polymers, carboxymethylcellulose sodium/calcium, hydroxpropyl methyl cellulose phthalate, shellac, effervescent mixtures such as bicarbonates in combination with one or more acids, e.g. citric acid or tartaric acid, or mixtures thereof. Preferably, the at least one disintegrant is sodium croscarmellose. The at least one disintegrant can be also a superdisintegrant. The superdisintegrant(s) that may be used in the method of the present invention generally are those well-known in the art. Exemplary superdisintegrants include but are not limited to sodium croscarmellose, insoluble cross-linked polyvinylpyrrolidones, sodium starch glycolate, and mixtures thereto.

If at least one disintegrant is added before and/or during and/or after step b), the at least one disintegrant is preferably added in an amount ranging from 0.3 to 10 wt.-%, preferably from 0.5 to 8 wt.-%, more preferably from 0.8 to about 5 wt.-%, and most preferably from 1 to about 5 wt.-%, based on the total dry weight of the magnesium ion-comprising material.

The at least one disintegrant may be added in dry form, or in the form of emulsions, dispersions, or solutions.

Thus, in one embodiment, the method for the production of granules comprising the magnesium ion-comprising material comprises the steps of

a) providing an aqueous suspension comprising a magnesium ion-comprising material;
b) homogenizing the aqueous suspension comprising a magnesium ion-comprising material of step a),
c) removing the liquid from the aqueous suspension comprising a magnesium ion-comprising material of step b) by means of spray drying for obtaining granules comprising the magnesium ion-comprising material, and
d) adding at least one disintegrant before and/or during and/or after step b) into the aqueous suspension.

Alternatively, the method may comprise a step d) of mechanically and/or physically disintegrating the aqueous suspension comprising a magnesium ion-comprising material before and/or during and/or after step b).

Such mechanical and/or physical disintegration may be carried out by any method known to the skilled person as being suitable for such purpose. For example, the mechanical and/or physical disintegration step d) may be carried out by ultrasonic probes and the like.

Thus, in one embodiment, the method for the production of granules comprising a magnesium ion-comprising material comprises the steps of

a) providing an aqueous suspension comprising a magnesium ion-comprising material;
b) homogenizing the aqueous suspension comprising a magnesium ion-comprising material of step a),
c) removing the liquid from the aqueous suspension comprising a magnesium ion-comprising material of step b) by means of spray drying for obtaining granules comprising a magnesium ion-comprising material, and
d) mechanically and/or physically disintegrating the aqueous suspension comprising a magnesium ion-comprising material before and/or during and/or after step b).

It is appreciated that further additives suitable for improving the mouthfeeling, palatability or controlled release such as polyols, e.g. mannitol, sorbitol and the like; carboxymethylcellulose, hydrocolloids, e.g. cellulos and its derivates, microcrystalline cellulose and the like; sugars or ground calcium carbonate (GCC) may be added before and/or during and/or after step b), preferably before or after step b), most preferably after step b).

Such additives, if added, are preferably added in an amount ranging from 0.3 to 40 wt.-%, preferably from 0.5 to 30 wt.-%, more preferably from 1 to about 25 wt.-% based on the total dry weight of the magnesium ion-comprising material.

According to step c) of the present invention the liquid is removed from the aqueous suspension comprising a magnesium ion-comprising material of step b) by means of spray drying for obtaining granules comprising a magnesium ion-comprising material.

The spray drying equipment may be selected from the conventionally used ones for spray drying purposes. Thus, the spray dryer may be selected from the group comprising rotary atomizer, fountain nozzle, bi-fluid nozzle, pressure nozzle, combi-nozzle, and the like. Preferably, spray-drying step c) is carried out by using a rotary atomizer or a bi-fluid nozzle. If homogenizing step b) is carried out by milling, the spray dryer may be selected from the conventionally used ones for spray drying, e.g. the spray dryer may be selected from the group comprising rotary atomizer, fountain nozzle, bi-fluid nozzle, pressure nozzle, combi-nozzle, and the like. As regards the fountain nozzle, it is to be noted that it may be also referred to as a pressure nozzle which is run in a fountain (or co-current) mode. In one embodiment, homogenizing step b) is carried out by milling and spray-drying step c) is carried out by using a rotary atomizer. It is appreciated that different conditions are to be set for the different spray drying techniques in order to achieve the desired granules. However, the skilled person knows how to adapt such conditions for the different spray drying techniques.

For example, if a pressure nozzle is used, the spray drying in step c) is carried out at

a) a feed pressure ranging from 0.1 to 300 bar, preferably from 1 to 100 bar, more preferably from 1 to < 50 bar, and most preferably from 1 to 25 bar, and/or
b) a temperature measured as inlet temperature ranging from 120 to 950°C, preferably from 175 to 700°C, and most preferably from 180 to 550°C.

In one embodiment, if a pressure nozzle is used, the spray drying in step c) is carried out at

a) feed a pressure ranging from 0.1 to 300 bar, preferably from 1 to 100 bar, more preferably from 1 to < 50 bar, and most preferably from 1 to 25 bar, or
b) a temperature measured as inlet temperature ranging from 120 to 950°C, preferably from 175 to 700°C, and most preferably from 180 to 550°C.

Preferably, if a pressure nozzle is used, the spray drying in step c) is carried out at

a) a feed pressure ranging from 0.1 to 300 bar, preferably from 1 to 100 bar, more preferably from 1 to < 50 bar, and most preferably from 1 to 25 bar, and
b) a temperature measured as inlet temperature ranging from 120 to 950°C, preferably from 175 to 700°C, and most preferably from 180 to 550°C.

In one embodiment, if a bi-fluid nozzle is used, the spray drying in step c) is carried out at

a) a feed pressure ranging from 0.1 to 300 bar, preferably from 1 to 100 bar, more preferably from 1 to < 50 bar, and most preferably from 1 to 25 bar, e.g. from 7 to 25 bar, and/or
b) an orifice diameter ranging from 0.8 to 1.8 mm, preferably from 0.9 to 1.6 mm, and most preferably from 1.05 to 1.5 mm, and/or
c) a temperature measured as inlet temperature ranging from 120 to 950°C, preferably from 175 to 700°C, and most preferably from 180 to 550°C, and/or
d) an air pressure to the nozzle from 1 to 7 bar, preferably from 1.5 to 6.5 bar and most preferably from 2 to 6 bar.

For example, if a bi-fluid nozzle is used, the spray drying in step c) is carried out at

a) a feed pressure ranging from 0.1 to 300 bar, preferably from 1 to 100 bar, more preferably from 1 to < 50 bar, and most preferably from 1 to 25 bar, e.g. from 7 to 25 bar, or
b) an orifice diameter ranging from 0.8 to 1.8 mm, preferably from 0.9 to 1.6 mm, and most preferably from 1.05 to 1.5 mm, or
c) a temperature measured as inlet temperature ranging from 120 to 950°C, preferably from 175 to 700°C, and most preferably from 180 to 550°C, or
d) an air pressure to the nozzle from 1 to 7 bar, preferably from 1.5 to 6.5 bar and most preferably from 2 to 6 bar.

Alternatively, if a bi-fluid nozzle is used, the spray drying in step c) is carried out at

a) a feed pressure ranging from 0.1 to 300 bar, preferably from 1 to 100 bar, more preferably from 1 to < 50 bar, and most preferably from 1 to 25 bar, e.g. from 7 to 25 bar, and
b) an orifice diameter ranging from 0.8 to 1.8 mm, preferably from 0.9 to 1.6 mm, and most preferably from 1.05 to 1.5 mm, and
c) a temperature measured as inlet temperature ranging from 120 to 950°C, preferably from 175 to 700°C, and most preferably from 180 to 550°C, and
d) an air pressure to the nozzle from 1 to 7 bar, preferably from 1.5 to 6.5 bar and most preferably from 2 to 6 bar.

It is appreciated that bi-fluid nozzles are well known in the art and include for example combi nozzles of GEA-Niro, Denmark.
In one embodiment, if a rotary atomizer is used, the spray drying in step c) is carried out at

a) a feed pressure ranging from 0.5 to 8 bar, preferably from 1 to 6.5 bar, and most preferably from 1 to 4.5 bar, e.g. from 1.5 to 4.5 bar, and/or
b) a speed of the rotary wheel of ≤ 11 000, preferably from 8 000 to 11 000 rpm, more preferably from 9 000 to 10 000 rpm (at a wheel diameter of d = 150 mm and/or a velocity of 73m/sec), and/or
c) a temperature measured as inlet temperature ranging from 120 to 950°C, preferably from 175 to 700°C, and most preferably from 180 to 550°C.

For example, if a rotary atomizer is used, the spray drying in step c) is carried out at

a) a feed pressure ranging from 0.5 to 8 bar, preferably from 1 to 6.5 bar, and most preferably from 1 to 4.5 bar, e.g. from 1.5 to 4.5 bar, or
b) a speed of the rotary wheel of ≤ 11 000, preferably from 8 000 to 11 000 rpm, more preferably from 9 000 to 10 000 rpm (at a wheel diameter of d = 150 mm and/or a velocity of 73m/sec), or
c) a temperature measured as inlet temperature ranging from 120 to 950°C, preferably from 175 to 700°C, and most preferably from 180 to 550°C.

Alternatively, if a rotary atomizer is used, the spray drying in step c) is carried out at

a) a pressure ranging from 0.5 to 8 bar, preferably from 1 to 6.55 bar, and most preferably from 1 to 4.5 bar, e.g. from 1.5 to 4.5 bar, and
b) a speed of the rotary wheel of ≤ 11 000, preferably from 8 000 to 11 000 rpm, more preferably from 9 000 to 10 000 rpm (at a wheel diameter of d = 150 mm and/or a velocity of 73m/sec), and
c) a temperature measured as inlet temperature ranging from 120 to 950°C, preferably from 175 to 700°C, and most preferably from 180 to 550°C.

In one embodiment, if a fountain nozzle is used, the spray drying in step c) is carried out at

a) a feed pressure ranging from 8 to 60 bar, preferably from 10 to 25 bar, and most preferably from 11 to 18 bar, and/or
b) a temperature measured as inlet temperature ranging from 120 to 950°C, preferably from 175 to 700°C, and most preferably from 180 to 550°C.

For example, if a fountain nozzle is used, the spray drying in step c) is carried out at

a) a feed pressure ranging from 8 to 60 bar, preferably from 10 to 25 bar, and most preferably from 11 to 18 bar, or
b) a temperature measured as inlet temperature ranging from 120 to 950°C, preferably from 175 to 700°C, and most preferably from 180 to 550°C.

Alternatively, if a fountain nozzle is used, the spray drying in step c) is carried out at

a) a pressure ranging from 8 to 60 bar, preferably from 10 to 25 bar, and most preferably from 11 to 18 bar, and
b) a temperature measured as inlet temperature ranging from 120 to 950°C, preferably from 175 to 700°C, and most preferably from 180 to 550°C.

The granules obtained in step c) are preferably in a dry form, i.e. a free flowing form.

The term "dry" granules is understood to be a material having less than 4 % by weight of water relative to the granule weight. The % water may be determined by heating the granules to 105°C in a drying chamber using the method according to ISO 787-2.

The granules obtained by the present process have a favourable bulk density. Thus, the present invention refers in another aspect to granules comprising a magnesium ion-comprising material, wherein the granules have a bulk density ranging from 0.1 to 0.70 g/mL. Preferably, the granules comprise a magnesium ion-comprising material, wherein the granules have a bulk density ranging from 0.1 to 0.70 g/mL. More preferably, the granules comprise a magnesium carbonate-comprising material, wherein the granules have a bulk density ranging from 0.1 to 0.70 g/mL. Most preferably, the granules comprise a surface-reacted magnesium carbonate-comprising material, wherein the granules have a bulk density ranging from 0.1 to 0.70 g/mL. For example, the granules comprise surface-reacted synthetic (or precipitated) hydromagnesite ($Mg_5(CO_3)_4(OH)_2 \cdot 4H_2O$), wherein the granules have a bulk density ranging from 0.1 to 0.70 g/mL.

For example, the granules have a bulk density ranging from 0.12 to 0.65 g/mL, more preferably from 0.20 to 0.60 g/mL and most preferably from 0.15 to 0.50 g/mL.

It is appreciated that the granules preferably have a very specific particle size distribution that can be adjusted according to the process used.

In particular, the granules have

a) a volume particle size $d_{90}$ of from 15 to 500 $\mu$m, as measured dry at 0.1 bar dispersion pressure by laser diffraction, and

b) a volume median particle size $d_{50}$ of from 5 to 300 $\mu$m, as measured dry at 0.1 bar dispersion pressure by laser diffraction, and

c) a volume particle size $d_{10}$ of 1 to 100 $\mu$m, as measured dry at 0.1 bar dispersion pressure by laser diffraction.

Preferably, the granules have

a) a volume particle size $d_{90}$ of from 20 to 400 $\mu$m, as measured dry at 0.1 bar dispersion pressure by laser diffraction, and

b) a volume median particle size $d_{50}$ of from 8 to 200 $\mu$m, as measured dry at 0.1 bar dispersion pressure by laser diffraction, and

c) a volume particle size $d_{10}$ of from 2 to 70 $\mu$m, as measured dry at 0.1 bar dispersion pressure by laser diffraction.

Most preferably, the granules have

a) a volume particle size $d_{90}$ of from 30 to 250 $\mu$m, as measured dry at 0.1 bar dispersion pressure by laser diffraction, and

b) a volume median particle size $d_{50}$ of from 10 to 150 $\mu$m, as measured dry at 0.1 bar dispersion pressure by laser diffraction, and

c) a volume particle size $d_{10}$ of from 4 to 50 $\mu$m, as measured dry at 0.1 bar dispersion pressure by laser diffraction.

Furthermore, the granules preferably have a BET specific surface area in the range from 20 to 90 $m^2$/g, preferably from 30 to 80 $m^2$/g, and most preferably from 40 to 70 $m^2$/g, measured using nitrogen and the BET method according to ISO 9277:2010.

Thus, the granules preferably have

a) a volume particle size $d_{90}$ of from 15 to 500 $\mu$m, preferably from 20 to 400 $\mu$m, and most preferably from 30 to 250 $\mu$m, as measured dry at 0.1 bar dispersion pressure by laser diffraction, and

b) a volume median particle size $d_{50}$ of from 5 to 300 $\mu$m, preferably from 8 to 200 $\mu$m, and most preferably from 10 to 150 $\mu$m, as measured dry at 0.1 bar dispersion pressure by laser diffraction, and

c) a volume particle size $d_{10}$ of from 1 to 100 $\mu$m, preferably from 2 to 70 $\mu$m, and most preferably from 4 to 50 $\mu$m, as measured dry at 0.1 bar dispersion pressure by laser diffraction, and

d) a BET specific surface area in the range from 20 to 90 $m^2$/g, preferably from 30 to 80 $m^2$/g, and most preferably from 40 to 70 $m^2$/g, measured using nitrogen and the BET method according to ISO 9277:2010.

Additionally or alternatively, the granules have a spherical shape. A "spherical shape" in the meaning of the present invention refers to a granule that has almost the same diameter on all axis in a three-dimensional space.

Thus, the granules preferably have

a) a volume particle size $d_{90}$ of from 15 to 500 $\mu$m, preferably from 20 to 400 $\mu$m, and most preferably from 30 to

250 $\mu$m, as measured dry at 0.1 bar dispersion pressure by laser diffraction, and
b) a volume median particle size $d_{50}$ of from 5 to 300 $\mu$m, preferably from 8 to 200 $\mu$m, and most preferably from 10 to 150 $\mu$m, as measured dry at 0.1 bar dispersion pressure by laser diffraction, and
c) a volume particle size $d_{10}$ of from 1 to 100 $\mu$m, preferably from 2 to 70 $\mu$m, and most preferably from 4 to 50 $\mu$m, as measured dry at 0.1 bar dispersion pressure by laser diffraction, and
d) a BET specific surface area in the range from 20 to 90 $m^2$/g, preferably from 30 to 80 $m^2$/g, and most preferably from 40 to 70 $m^2$/g, measured using nitrogen and the BET method according to ISO 9277:2010, and/or
e) a spherical shape.

For example, the granules preferably have

a) a volume particle size $d_{90}$ of from 15 to 500 $\mu$m, preferably from 20 to 400 $\mu$m, and most preferably from 30 to 250 $\mu$m, as measured dry at 0.1 bar dispersion pressure by laser diffraction, and
b) a volume median particle size $d_{50}$ of from 5 to 300 $\mu$m, preferably from 8 to 200 $\mu$m, and most preferably from 10 to 150 $\mu$m, as measured dry at 0.1 bar dispersion pressure by laser diffraction, and
c) a volume particle size $d_{10}$ of from 1 to 100 $\mu$m, preferably from 2 to 70 $\mu$m, and most preferably from 4 to 50 $\mu$m, as measured dry at 0.1 bar dispersion pressure by laser diffraction, and
d) a BET specific surface area in the range from 20 to 90 $m^2$/g, preferably from 30 to 80 $m^2$/g, and most preferably from 40 to 70 $m^2$/g, measured using nitrogen and the BET method according to ISO 9277:2010, or
e) a spherical shape.

Alternatively, the granules preferably have

a) a volume particle size $d_{90}$ of from 15 to 500 $\mu$m, preferably from 20 to 400 $\mu$m, and most preferably from 30 to 250 $\mu$m, as measured dry at 0.1 bar dispersion pressure by laser diffraction, and
b) a volume median particle size $d_{50}$ of from 5 to 300 $\mu$m, preferably from 8 to 200 $\mu$m, and most preferably from 10 to 150 $\mu$m, as measured dry at 0.1 bar dispersion pressure by laser diffraction, and
c) a volume particle size $d_{10}$ of from 1 to 100 $\mu$m, preferably from 2 to 70 $\mu$m, and most preferably from 4 to 50 $\mu$m, as measured dry at 0.1 bar dispersion pressure by laser diffraction, and
d) a BET specific surface area in the range from 20 to 90 $m^2$/g, preferably from 30 to 80 $m^2$/g, and most preferably from 40 to 70 $m^2$/g, measured using nitrogen and the BET method according to ISO 9277:2010, and
e) a spherical shape.

It is further to be noted that the granules show a favorable stability. In particular, the granules show a stability that is higher compared to granules obtained by the same method but missing the step of homogenizing the aqueous suspension comprising the magnesium ion-comprising material. For example, the granules have a stability determined by the ratio $d_{50}$ for (0.5 bar) vs. (0.1 bar) of $\geq$ 15, more preferably $\geq$ 20, even more preferably $\geq$ 25 and most preferably $\geq$ 30, like in the range from 30 to 90. Additionally or alternatively, the granules have a stability determined by the ratio $d_{50}$ for (1.5 bar) vs. (0.1 bar) of $\geq$ 5, more preferably $\geq$ 7, even more preferably $\geq$ 10 and most preferably $\geq$ 12, like in the range from 12 to 90 or 12 to 80.
It is further preferred that the granules have a stability determined by a ratio $d_{50}$ for (0.5 bar) vs. (0.1 bar) of $\geq$ 15 %, more preferably $\geq$ 20 %, even more preferably $\geq$ 25 % and most preferably $\geq$ 30 %, like in the range from 30 to 80 %. Additionally or alternatively, the granules have a stability determined by a ratio $d_{50}$ for (1.5 bar) vs. (0.1 bar) of $\geq$ 5 %, more preferably $\geq$ 7 %, even more preferably $\geq$ 10 % and most preferably $\geq$ 12 %, like in the range from 12 to 90 % or 12 to 80 %.
Additionally, the granules have an intra-granular specific pore volume within the range from 0.15 to 2.75 $cm^3$/g, preferably from 0.30 to 2.50 $cm^3$/g, and most preferably from 0.40 to 2.00 $cm^3$/g, calculated from a mercury intrusion porosimetry measurement.
The granules comprise particles of a magnesium ion-comprising material preferably having a BET specific surface area of from 1 $m^2$/g to 200 $m^2$/g, preferably 2 $m^2$/g to 150 $m^2$/g, more preferably 20 $m^2$/g to 140 $m^2$/g, most preferably 40 $m^2$/g to 70 $m^2$/g, measured using nitrogen and the BET method according to ISO 9277:2010.
It is further preferred that the granules comprise particles of a magnesium ion-comprising material having a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, measured by using laser diffraction.
In view of the above, the granules comprise particles of a magnesium ion-comprising material having a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and/or

a) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction.

For example, the granules comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, or
b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction.

Preferably, the granules comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and
b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction.

Additionally or alternatively, the granules comprise particles of a magnesium ion-comprising material having a BET specific surface area in the range from 10 to 100 m$^2$/g, preferably from 12 to 70 m$^2$/g, and most preferably from 17 to 60 m$^2$/g, measured using nitrogen and the BET method according to ISO 9277:2010.
Thus, the granules preferably comprise particles of a magnesium ion-comprising material having a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and/or

a) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, and/or
b) a BET specific surface area in the range from 10 to 100 m$^2$/g, preferably from 12 to 70 m$^2$/g, and most preferably from 17 to 60 m$^2$/g, measured using nitrogen and the BET method according to ISO 9277:2010.

For example, the granules comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and
b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, and/or
c) a BET specific surface area in the range from 10 to 100 m$^2$/g, preferably from 12 to 70 m$^2$/g, and most preferably from 17 to 60 m$^2$/g, measured using nitrogen and the BET method according to ISO 9277:2010.

In one embodiment, the granules comprise particles of a magnesium ion-comprising material having a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and

a) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, or
b) a BET specific surface area in the range from 10 to 100 m$^2$/g, preferably from 12 to 70 m$^2$/g, and most preferably from 17 to 60 m$^2$/g, measured using nitrogen and the BET method according to ISO 9277:2010.

Preferably, the granules comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and
b) a volume top cut particle size $d_{98}$ in the range from 2 to 150 $\mu$m, preferably from 4 to 100 $\mu$m, more preferably

from 6 to 80 $\mu$m, even more preferably from 8 to 60 $\mu$m and most preferably from 10 to 40 $\mu$m, as determined by laser diffraction, and
c) a BET specific surface area in the range from 10 to 100 m$^2$/g, preferably from 12 to 70 m$^2$/g, and most preferably from 17 to 60 m$^2$/g, measured using nitrogen and the BET method according to ISO 9277:2010.

Additionally or alternatively, the granules preferably comprise particles of a magnesium ion-comprising material having an intra-particle intruded specific pore volume in the range from 0.9 to 2.3 cm$^3$/g, preferably from 1.2 to 2.1 cm$^3$/g, and most preferably from 1.5 to 2.0 cm$^3$/g, calculated from mercury porosimetry measurement.
For example, the granules comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and/or
b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, and/or
c) an intra-particle intruded specific pore volume in the range from 0.9 to 2.3 cm$^3$/g, preferably from 1.2 to 2.1 cm$^3$/g, and most preferably from 1.5 to 2.0 cm$^3$/g, calculated from mercury porosimetry measurement.

In one embodiment, the granules comprise particles of a magnesium ion-comprising material having a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and

a) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, and/or
b) an intra-particle intruded specific pore volume in the range from 0.9 to 2.3 cm$^3$/g, preferably from 1.2 to 2.1 cm$^3$/g, and most preferably from 1.5 to 2.0 cm$^3$/g, calculated from mercury porosimetry measurement.

For example, the granules comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and
b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, or
c) an intra-particle intruded specific pore volume in the range from 0.9 to 2.3 cm$^3$/g, preferably from 1.2 to 2.1 cm$^3$/g, and most preferably from 1.5 to 2.0 cm$^3$/g, calculated from mercury porosimetry measurement.

Preferably, the granules comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, and
b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction, and
c) an intra-particle intruded specific pore volume in the range from 0.9 to 2.3 cm$^3$/g, preferably from 1.2 to 2.1 cm$^3$/g, and most preferably from 1.5 to 2.0 cm$^3$/g, calculated from mercury porosimetry measurement.

In a preferred embodiment, the granules thus comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and/or
b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and/or
c) a BET specific surface area in the range from 10 to 100 m$^2$/g, preferably from 12 to 70 m$^2$/g, and most preferably from 17 to 60 m$^2$/g, measured using nitrogen and the BET method according to ISO 9277:2010; and/or

d) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

Preferably, the granules comprise particles of a magnesium ion-comprising material having

a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and
b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and
c) a BET specific surface area in the range from 10 to 100 $m^2/g$, preferably from 12 to 70 $m^2/g$, and most preferably from 17 to 60 $m^2/g$, measured using nitrogen and the BET method according to ISO 9277:2010; and/or
d) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

More preferably, the granules comprise particles of a magnesium ion-comprising material having a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and

a) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and
b) a BET specific surface area in the range from 10 to 100 $m^2/g$, preferably from 12 to 70 $m^2/g$, and most preferably from 17 to 60 $m^2/g$, measured using nitrogen and the BET method according to ISO 9277:2010; and
c) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

In one embodiment, the granules comprise at least one disintegrant or any compound that may act as disintegrant. For example, the at least one disintegrant is selected from the group comprising sodium croscarmellose, modified cellulose gums, insoluble cross-linked polyvinylpyrrolidones, starches, modified starches, starch glycolates such as sodium starch glycolate, micro crystalline cellulose, pregelatinized starch, sodium carboxymethyl starch, low-substituted hydroxypropyl cellulose, homopolymers of N-vinyl-2-pyrrolidone, alkyl-, hydroxyalkyl-, carboxyalkyl-cellulose esters, alginic acid, microcrystalline cellulose and its polymorphic forms, ion exchange resins, gums, chitin, chitosan, clays, gellan gum, crosslinked polacrillin copolymers, agar, gelatine, dextrines, acrylic acid polymers, carboxymethylcellulose sodium/calcium, hydroxpropyl methyl cellulose phthalate, shellac, effervescent mixtures such as bicarbonates in combination with one or more acids, e.g. citric acid or tartaric acid, or mixtures thereof.

If present, the granules comprise the at least one disintegrant (or any compound that may act as disintegrant) in an amount ranging from 0.25 to 35 wt.-%, preferably of from 0.5 to 15 wt.-%, more preferably of from 0.5 to 10 wt.-%, even more preferably of from 0.7 to 10 wt.-%, most preferably of from 0.8 to 10 wt.-%, based on the total dry weight of the granules. In one embodiment, the granules comprise the at least one disintegrant (or any compound that may act as disintegrant) in an amount ranging from 0.25 to 35 wt.-%, preferably of from 0.5 to 15 wt.-%, more preferably of from 0.5 to 10 wt.-%, even more preferably of from 1.0 to 10 wt.-%, most preferably of from 1.5 to 10 wt.-%, based on the total dry weight of the granules. It is specifically preferred that the granules comprise the at least one disintegrant (or any compound that may act as disintegrant) in an amount ranging from 0.8 to 8 wt.-%, preferably of from 0.8 to 6 wt.-%, more preferably of from 0.8 to 5 wt.-%, and most preferably of from 0.8 to 4 wt.-%, based on the total dry weight of the granules.

It is appreciated that the granules are preferably obtained by a method as defined herein.

Thus, the granules are preferably obtained by a method comprising the steps of

a) providing an aqueous suspension comprising a magnesium ion-comprising material;
b) homogenizing the aqueous suspension comprising a magnesium ion-comprising material of step a), and
c) removing the liquid from the aqueous suspension comprising a magnesium ion-comprising material of step b) by means of spray drying for obtaining granules comprising a magnesium ion-comprising material.

If the granules comprise at least one disintegrant (or any compound that may act as disintegrant), the granules are preferably obtained by a method comprising the steps of

a) providing an aqueous suspension comprising a magnesium ion-comprising material;

b) homogenizing the aqueous suspension comprising a magnesium ion-comprising material of step a),

c) removing the liquid from the aqueous suspension comprising a magnesium ion-comprising material of step b) by means of spray drying for obtaining granules comprising a magnesium ion-comprising material, and

d) adding at least one disintegrant (or any compound that may act as disintegrant) before and/or during and/or after step b) into the aqueous suspension.

Additionally or alternatively, the granules are subjected to a treatment with the at least one active ingredient and/or inactive precursor thereof such that the at least one active ingredient and/or inactive precursor thereof is substantially only present on the outer surface of the granules.

The term "active ingredient" in the meaning of the present invention refers to a substance having a specific effect in an organism and causing a specific reaction in humans, animals, microorganisms and/or plants.

It is preferred that the at least one active ingredient and/or inactive precursor thereof is/are provided in liquid form.

The term "liquid" in the meaning of the present invention refers to a non-gaseous fluid composition, comprising or consisting of the at least one active ingredient and/or inactive precursor thereof, which is readily flowable at the pressure conditions and temperature of use, i.e. the pressure and temperature at which the granules are mixed with the at least one active ingredient and/or inactive precursor thereof.

Thus, it is appreciated that the at least one active ingredient and/or inactive precursor thereof can be liquid in a temperature range from 5 to 200°C, preferably from 10 to 120°C and most preferably from 10 to 100°C. For example, the at least one active ingredient and/or inactive precursor thereof can be liquid in a temperature range from 5 to 200°C, preferably from 10 to 120°C and most preferably from 10 to 100°C at ambient pressure conditions, i.e. at atmospheric pressure. Alternatively, the at least one active ingredient and/or inactive precursor thereof can be liquid in a temperature range from 5 to 200°C, preferably from 10 to 120°C and most preferably from 10 to 100°C at reduced pressure conditions, e.g. a pressure of from 100 to 700 mbar.

In one embodiment, the at least one active ingredient and/or inactive precursor thereof is/are liquid at ambient temperature and pressure conditions, e.g., at room temperature, such as from about 5 to 35°C, preferably from 10 to 30°C and most preferably from 15 to 25°C, and at atmospheric pressure.

Alternatively, the at least one active ingredient and/or inactive precursor thereof is/are molten at the temperature of use, e.g. from about 35 to 200°C, preferably from 45 to 120°C and most preferably from 55 to 100°C, and at ambient pressure conditions, i.e. at atmospheric pressure, or at reduced pressure conditions, e.g. a pressure of from 100 to 700 mbar.

Alternatively, the at least one active ingredient and/or inactive precursor thereof is/are dissolved in a solvent. That is to say, the at least one active ingredient and/or inactive precursor thereof and the solvent form a system in which no discrete solid particles are observed in the solvent and thus form a "solution".

In one embodiment of the present invention, the solvent is selected from the group comprising water, methanol, ethanol, n-butanol, isopropanol, n-propanol, acetone, dimethylsulphoxide, dimethylformamide, tetrahydrofurane, vegetable oils and the derivatives thereof, animal oils and the derivatives thereof, molten fats and waxes, and mixtures thereof. Preferably, the solvent is water, ethanol and/or acetone. More preferably, the solvent is water.

For example, the at least one active ingredient and/or inactive precursor thereof may be a chiral compound. Thus, the at least one active ingredient and/or inactive precursor thereof encompass the (R)-enantiomer, (S)-enantiomer and mixtures thereof, e.g. the racemic mixture.

Additionally or alternatively, the at least one active ingredient and/or inactive precursor thereof may be an isomeric compound. Thus, the at least one active ingredient and/or inactive precursor thereof encompass the (Z)-isomer, (E)-isomer and mixtures thereof. For example, if it is stated that the active ingredient is cinnamaldehyde, the cinnamaldehyde may be present as (Z)-cinnamaldehyde and/or (E)-cinnamaldehyde.

For example, the at least one active ingredient and/or inactive precursor thereof is selected from the group comprising fragrances, flavours, herbal extracts and oils, fruit extracts and oils, nutrients, trace minerals, repellents, food, cosmetics, flame retardants, enzymes, macromolecules, pesticides, fertilizers, preserving agents, antioxidants, reactive chemicals, pharmaceutical and/or nutraceutical and/or veterinary active agents or pharmaceutical and/or nutraceutical and/or veterinary inactive precursors of synthetic origin, semi-synthetic origin, natural origin thereof, and mixtures thereof.

Fragrances are preferably alcohols, aldehydes and/or ketones having a molecular weight of at least about 100 g/mol and which are useful in imparting an odour, fragrance, essence, or scent either alone or in combination with other fragrances. For example, the fragrance can be selected from the group comprising 2,4-dimethyl-3-cyclohexene-1-methanol (floralol), 2,4-dimethyl cyclohexane methanol (dihydro floralol), 5,6-dimethyl-1-methylethenylbicyclo[2.2.1]hept-5-ene-2-methanol (arbozol), $\alpha,\alpha$,-4-trimethyl-3-cyclohexen-1-methanol ($\alpha$-terpineol), 2,4,6-trimethyl-3-cyclohexene-1-methanol (isocyclo geraniol), 4-(1-methylethyl)cyclohexane methanol (mayol), $\alpha$-3,3-trimethyl-2-norborane methanol, 1,1-dimethyl-1-(4-methylcyclohex-3-enyl)methanol, 2-phenylethanol, 2-cyclohexyl ethanol, 2-(o-methylphenyl)-ethanol, 2-(m-methylphenyl)ethanol, 2-(p-methylphenyl)ethanol, 6,6-dimethylbicyclo-[3.1.1]hept-2-ene-2-ethanol (nopol), 2-(4-methylphenoxy)-ethanol, 3,3-dimethyl-$\Delta^2$-$\beta$-norbornane ethanol (patchomint), 2-methyl-2-cyclohexylethanol, 1-(4-isopropylcyclohexyl)-ethanol, 1-phenylethanol, 1,1-dimethyl-2-phenylethanol, 1,1-dimethyl-2-(4-methyl-phenyl)ethanol, 1-phenylpropanol, 3-phenylpropanol, 2-phenylpropanol (Hydrotropic Alcohol), 2-(cyclododecyl)propan-1-ol (Hydroxy-ambran), 2,2-dimethyl-3-(3-methylphenyl)-propan-1-ol (Majantol), 2-methyl-3-phenylpropanol, 3-phenyl-2-propen-1-ol (cinnamyl alcohol), 2-methyl-3-phenyl-2-propen-1-ol (methylcinnamyl alcohol), $\alpha$-n-pentyl-3-phenyl-2-propen-1-ol ($\alpha$-amylcinnamyl alcohol), ethyl-3-hydroxy-3-phenyl propionate, 2-(4-methylphenyl)-2-propanol, 3-(4-methylcyclohex-3-ene)butanol, 2-methyl-4-(2,2,3-trimethyl-3-cyclopenten-1-yl)butanol, 2-ethyl-4-(2,2,3-trimethyl-cyclopent-3-enyl)-2-buten-1-ol, 3-methyl-2-buten-1-ol (prenol), 2-methyl-4-(2,2,3-trimethyl-3-cyclopenten-1-yl)-2-buten-1-ol, ethyl 3-hydroxybutyrate, 4-phenyl-3-buten-2-ol, 2-methyl-4-phenylbutan-2-ol, 4-(4-hydroxyphenyl)butan-2-one, 4-(4-hydroxy-3-methoxyphenyl)-butan-2-one, 3-methyl-pentanol, 3-methyl-3-penten-1-ol, 1-(2-propenyl)cyclopentan-1-ol (plinol), 2-methyl-4-phenylpentanol (Pamplefleur), 3-methyl-5-phenylpentanol (Phenoxanol), 2-methyl-5-phenylpentanol, 2-methyl-5-(2,3-dimethyltricyclo[2.2.1.0.sup.(2,6)]hept-3-yl)-2-penten-1-ol (santalol), 4-methyl-1-phenyl-2-pentanol, 5-(2,2,3-trimethyl-3-cyclopentenyl)-3-methylpentan-2-ol (sandalore), (1-methyl-bicyclo[2.1.1]hepten-2-yl)-2-methylpent-1-en-3-ol, 3-methyl-1-phenylpentan-3-ol, 1,2-dimethyl-3-(1-methylethenyl)cyclopentan-1-ol, 2-isopropyl-5-methyl-2-hexenol, cis-3-hexen-1-ol, trans-2-hexen-1-ol, 2-isoproenyl-4-methyl-4-hexen-1-ol (Lavandulol), 2-ethyl-2-prenyl-3-hexenol, 1-hydroxymethyl-4-isopropenyl-1-cyclohexene (Dihydrocuminyl alcohol), 1-methyl-4-isopropenylcyclohex-6-en-2-ol (carvenol), 6-methyl-3-isopropenylcyclohexan-1-ol (dihydrocarveol), 1-methyl-4-iso-propenylcyclohexan-3-ol, 4-isopropyl-1-methylcyclohexan-3-ol, 4-tert-butylcyclo-hexanol, 2-tert-butylcyclohexanol, 2-tert-butyl-4-methylcyclohexanol (rootanol), 4-isopropyl-cyclohexanol, 4-methyl-1-(1-methylethyl)-3-cyclohexen-1-ol, 2-(5,6,6-trimethyl-2-norbornyl)cyclohexanol, isobornylcyclohexanol, 3,3,5-trimethylcyclohexanol, 1-methyl-4-isopropylcyclohexan-3-ol, 1-methyl-4-isopropylcyclohexan-8-ol (dihydroterpineol), 1,2-dimethyl-3-(1-methylethyl)cyclohexan-1-ol, heptanol, 2,4-dimethylheptan-1-ol, 6-heptyl-5-hepten-2-ol (isolinalool), 2,4-dimethyl-2,6-heptandienol, 6,6-dimethyl-2-oxymethyl-bicyclo[3.1.1]hept-2-ene (myrtenol), 4-methyl-2,4-heptadien-1-ol, 3,4,5,6,6-pentamethyl-2-heptanol, 3,6-dimethyl-3-vinyl-5-hepten-2-ol, 6,6-dimethyl-3-hydroxy-2-methylenebicyclo[3.1.1]heptane, 1,7,7-trimethylbicyclo[2.2.1]heptan-2-ol, 2,6-dimethylheptan-2-ol (dimetol), 2,6,6-trimethylbicyclo[1,3.3]heptan-2-ol, octanol, 2-octenol, 2-methyloctan-2-ol, 2-methyl-6-methylene-7-octen-2-ol (myrcenol), 7-methyloctan-1-ol, 3,7-dimethyl-6-octenol, 3,7-dimethyl-7-octenol, 3,7-dimethyl-6-octen-1-ol (citronellol), 3,7-dimethyl-2,6-octadien-1-ol (geraniol), 3,7-dimethyl-2,6-octadien-1-ol (nerol), 3,7-dimethyl-7-methoxyoctan-2-ol (osyrol), 3,7-dimethyl-1,6-octadien-3-ol (linalool), 3,7-dimethyloctan-1-ol (pelargol), 3,7-dimethyloctan-3-ol (tetrahydrolinalool), 2,4-octadien-1-ol, 3,7-dimethyl-6-octen-3-ol (dihydrolinalool), 2,6-dimethyl-7-octen-2-ol (dihydromyrcenol), 2,6-dimethyl-5,7-octadien-2-ol, 4,7-dimethyl-4-vinyl-6-octen-3-ol, 3-methyloctan-3-ol, 2,6-dimethyloctan-2-ol, 2,6-dimethyloctan-3-ol, 3,6-dimethyloctan-3-ol, 2,6-dimethyl-7-octen-2-ol, 2,6-dimethyl-3,5-octadien-2-ol (muguol), 3-methyl-1-octen-3-ol, 7-hydroxy-3,7-dimethyloctanal, 3-nonanol, 2,6-nonadien-1-ol, cis-6-nonen-1-ol, 6,8-dimethylnonan-2-ol, 3-(hydroxymethyl)-2-nonanone, 2-nonen-1-ol, 2,4-nonadien-1-ol, 3,7-dimethyl-1,6-nonadien-3-ol, decanol, 9-decenol, 2-benzyl-M-dioxa-5-ol, 2-decen-1-ol, 2,4-decadien-1-ol, 4-methyl-3-decen-5-ol, 3,7,9-trimethyl-1,6-decadien-3-ol (isobutyl linalool), undecanol, 2-undecen-1-ol, 10-undecen-1-ol, 2-dodecen-1-ol, 2,4-dodecadien-1-ol, 2,7,11-trimethyl-2,6,10-dodecatrien-1-ol (farnesol), 3,7,11-trimethyl-1,6,10,-dodecatrien-3-ol (nerolidol), 3,7,11,15-tetramethylhexadec-2-en-1-ol (phytol), 3,7,11,15-tetramethylhexadec-1-en-3-ol (iso phytol), benzyl alcohol, p-methoxy benzyl alcohol (anisyl alcohol), para-cymen-7-ol (cuminyl alcohol), 4-methyl benzyl alcohol, 3,4-methylenedioxy benzyl alcohol, methyl salicylate, benzyl salicylate, cis-3-hexenyl salicylate, n-pentyl salicylate, 2-phenylethyl salicylate, n-hexyl salicylate, 2-methyl-5-isopropylphenol, 4-ethyl-2-methoxyphenol, 4-allyl-2-methoxyphenol (eugenol), 2-methoxy-4-(1-propenyl)phenol (isoeugenol), 4-allyl-2,6-dimethoxy-phenol, 4-tert-butylphenol, 2-ethoxy-4-methylphenol, 2-methyl-4-vinylphenol, 2-isopropyl-5-methylphenol (thymol), pentyl-ortho-hydroxy benzoate, ethyl 2-hydroxybenzoate, methyl 2,4-dihydroxy-3,6-dimethylbenzoate, 3-hydroxy-5-methoxy-1-methylbenzene, 2-tert-butyl-4-methyl-1-hydroxybenzene, 1-ethoxy-2-hydroxy-4-propenylbenzene, 4-hydroxytoluene, 4-hydroxy-3-methoxybenzaldehyde, 2-ethoxy-4-hydroxybenzaldehyde, decahydro-2-naphthol, 2,5,5-trimethyl-octahydro-2-naphthol, 1,3,3-trimethyl-2-norbornanol (fenchol), 3a,4,5,6,7,7a-hexahydro-2,4-dimethyl-4,7-methano-1H-inden-5-ol, 3a,4,5,6,7,7a-hexahydro-3,4-dimethyl-4,7-methano-1H-inden-5-ol, 2-methyl-2-vinyl-5-(1-hydroxy-1-methylethyl)tetra-hydrofuran, $\beta$-caryophyllene alcohol, vanillin, ethyl vanillin, cinnamaldehyde, benzaldehyde, phenyl acetaldehyde, heptylaldehyde, octylaldehyde, decylaldehyde, undecylaldehyde, undecylenic aldehyde, dodecylaldehyde, tridecylaldehyde, methylnonyl aldehyde, didecylaldehyde, anisaldehyde, citronellal, citronellyloxyaldehyde, cyclamen aldehyde, $\alpha$-hexyl cinnamaldehyde, hydroxycitronellal, $\alpha$-methyl cinnamaldehyde, methylnonyl acetaldehyde, propylphenyl aldehyde, citral, perilla aldehyde, tolylaldehyde, tolylacetaldehyde, cuminaldehyde, LILIAL®, salicyl aldehyde, $\alpha$-amylcinnamaldehyde and heliotropin and mixtures thereof.

Various essential oils, herbal extracts and/or fruit extracts may also be used, preferably those with various medicinal or dietary supplement properties. Essential oils, herbal extracts and/or fruit extracts are generally extracts or aromatic plants, plant parts, fruit or fruit parts that can be used medicinally or for flavouring. Suitable herbal extracts and/or fruit extracts can be used singly or in various mixtures. Commonly used essential oils, herbal extracts and/or fruit extracts include Echinacea, Goldenseal, Calendula, Rosemary, Thyme, Kava Kava, Aloe, Blood Root, Grapefruit Seed Extract, Black Cohosh, Ginseng, Guarana, Cranberry, Ginko Biloba, St. John's Wort, Evening Primrose Oil, Yohimbe Bark, Green Tea, Ma Huang, Maca, Bilberry, Lutein, Ginger, eugenol-containing oils and combinations thereof.

A variety of nutrients may be used including virtually any vitamin, mineral and/or phytochemical. For example, vitamin A, vitamin B1, vitamin B6, vitamin B12, vitamin B2, vitamin B6, vitamin D, vitamin E, i.e. tocopheroles, vitamin K, thiamine, riboflavin, biotin, folic acid, niacin, pantothenic acid, Q10, alpha lipoic acid, dihydrolipoic acid, curcumin, xanthophylls, beta cryptoxanthin, lycopene, lutein, zeaxanthin, astaxanthin, beta-carotene, carotenes, mixed carotenoids, polyphenols, flavonoids, sodium, potassium, calcium, magnesium, sulphur, chlorine, choline, and/or phytochemicals such as carotenoids, chlorophyll, chlorophyllin, fibre, flavanoids, anthocyanins, cyaniding, delphinidin, malvidin, pelargonidin, peonidin, petunidin, flavanols, catechin, epicatechin, epigallocatechin, epigallocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof, may be used. Examples of nutrients that can be used as active ingredient(s) are set forth in U.S. Patent Application Publication Nos. 20030157213 A1, 20030206993 and 20030099741 A1 which are incorporated in their entirety herein by reference for all purposes.

In one embodiment, trace minerals can be used, e.g. manganese, zinc, copper, fluorine, molybdenum, iodine, cobalt, chromium, selenium, phosphorous, and combinations thereof

Enzymes can include but are not limited to coenzyme Q10, pepsin, phytase, trypsin, lipases, proteases, cellulases, lactase and combinations thereof.

Pesticides are preferably any known herbicide, insecticide, insect growth regulator, nematicide, termiticide, molluscicide, piscicide, avicide, rodenticide, predacide, bactericide, insect repellent, animal repellent, antimicrobial, fungicide, disinfectant (antimicrobial), and sanitizer known to the skilled person.

It is to be noted that the preserving agent may be any such compound known to the skilled person. For example, preserving agents may include, but are not limited to, phenoxyethanol, ethylhexylglycerin, parabens such as methyl paraben, ethyl paraben, propyl paraben, butyl paraben and mixtures thereof, benzalkonium chloride, chlorbutanol, benzyl alcohol, cetylpyridinium chloride, tartaric acid, lactic acid, malic acid, acetic acid, benzoic acid, sodium benzoate, sorbic acid, potassium sorbate and mixtures thereof.

Antioxidants are preferably selected from the group comprising butylhydroxyanisol (BHA), butylhydroxytoluol (BHT), gallate, carotinoid, polyphenols such as resveratrol, flavonoid and mixtures thereof, derivatives of polyphenols, tocopherol and salts thereof, betacarotin, ubichinon, tocotrienol, dihydroquercetin, antioxidants of natural origin and mixtures thereof. If the antioxidant is of natural origin, the antioxidant can be e.g. a conifer extract, pinus pinaster bark extract such as Pycnogenol® from Horphag, Switzerland, and/or emblica officinalis fruit extract such as Saberry® from Sabinsa corporation, Germany.

The pharmaceutically active agent or pharmaceutically inactive precursor thereof is preferably selected from the group comprising pharmaceutically active agent or pharmaceutically inactive precursor of synthetic origin, semi-synthetic origin, natural origin and combinations thereof.

Thus, a pharmaceutically active agent refers to pharmaceutically active agents which are of synthetic origin, semi-synthetic origin, natural origin and combinations thereof. Further, a pharmaceutically inactive precursor of the pharmaceutically active agent refers to pharmaceutically inactive precursors which are of synthetic origin, semi-synthetic origin, natural origin and combinations thereof and will be activated at a later stage to the respective pharmaceutically active agent.

The activation of such pharmaceutically inactive precursor is known to the skilled person and commonly in use, e.g. activation in the stomach and/or gastro-intestinal pathway- such as acidic activation or tryptic- or chimotryptic cleavage.

It lies within the understanding of the skilled person that the mentioned activation methods are of mere illustrative character and are not intended to be of limiting character.

It is to be noted that the pharmaceutically active agent or pharmaceutically inactive precursor thereof, may be any such compound known to the skilled person.

Pharmaceutically active agents thus include any compound that provides prophylactic and/or therapeutic properties when administered to humans and/or animals. Examples include, but are not limited to, pharmaceutical actives, therapeutic actives, veterinarian actives, nutraceuticals, and growth regulators.

The pharmaceutically active agent or pharmaceutically inactive precursor thereof can be an anti-inflammatory agent. Such agents may include, but are not limited to, non- steroidal anti-inflammatory agents or NSAIDs, such as propionic acid derivatives; acetic acid derivatives; fenamic acid derivatives; biphenylcarboxylic acid derivatives; and oxicams. All of these NSAIDs are fully described in U.S. Patent Number 4,985,459 to Sunshine et al., incorporated by reference herein in its entirety as to the description of such NSAIDs. Examples of useful NSAIDs include acetylsalicylic acid, ibuprofen, naproxen, benoxaprofen, flurbiprofen, fenoprofen, fenbufen, ketoprofen, indoprofen, pirprofen, carprofen, oxaprozin, pranoprofen, microprofen, tioxaprofen, suprofen, alminoprofen, tiaprofenic acid, fluprofen, bucloxic acid and mixtures thereof. Also useful are the steroidal anti-inflammatory drugs such as hydrocortisone and the like, and COX-2 inhibitors such as meloxicam, celecoxib, rofecoxib, valdecoxib, etoricoxib or mixtures thereof. Mixtures of any of the above anti-inflammatories may be used.

Other materials that can be used as pharmaceutically active agent or pharmaceutically inactive precursor thereof include commonly known mouth and throat products. These products include, but are not limited to, upper respiratory agents such as phenylephrine, diphenhydramine, dextromethorphan, bromhexine and chlorpheniramine, gastrointestinal agents such as famotidine, loperamide and simethicone, anti-fungals such as miconazole nitrate, antibiotics and analgesics such as ketoprofen and fluribuprofen.

The pharmaceutically active agent or pharmaceutically inactive precursor thereof may be also selected from sodium pyrosulphite, butylhydroxytoluene, butylated hydroxyanisole.

The pharmaceutically active agent or pharmaceutically inactive precursor thereof may be also selected from ephedrine, magaldrate, pseudoephedrine, sildenafil, xylocaine, benzalconium chloride, caffeine, phenylephrine, amfepramone, orlistat, sibutramine, acetaminophen, aspirin, glitazones, metformin, chlorpromazine, dimenhydrinat, domperidone, meclozine, metoclopramide, odansetron, prednisolone, promethazine, acrivastine, cetirizine, cinnarizine, clemastine, cyclizine, desloratadine, dexchlorpheniramine, dimenhydrinate, ebastine, fexofenadine, ibuprofen, levolevoproricin, loratadine, meclozine, mizolastine, promethazine, miconazole, chlorhexidine diacetate, fluoride, decapeptide KSL, aluminium fluoride, aminochelated calcium, ammonium fluoride, ammonium fluorosilicate, ammonium monofluorphosphate, calcium fluoride, calcium gluconate, calcium glycerophosphate, calcium lactate, calcium monofluorphosphate, calciumcarbonate, carbamide, cetyl pyridinium chloride, chlorhexidine, chlorhexidine digluconate, chlorhexidine chloride, chlorhexidine diacetate, CPP caseine phospho peptide, hexetedine, octadecentyl ammonium fluoride, potassium fluorosilicate, potassium chloride, potassium monofluorphosphate, sodium bi carbonate, sodium carbonate, sodium fluoride, sodium fluorosilicate, sodium monofluorphosphate, sodium tri polyphosphate, stannous fluoride, stearyl trihydroxyethyl propylenediamine dihydrofluoride, strontium chloride, tetra potassium pyrophosphate, tetra sodium pyrophosphate, tripotassium orthophosphate, trisodium orthophosphate, alginic acid, aluminium hydroxide, sodium bicarbonate, sildenafil, tadalafil, vardenafil, yohimbine, cimetidine, nizatidine, ranitidine, acetylsalicylic acid, clopidogrel, acetylcysteine, bromhexine, codeine, dextromethorphan, diphenhydramine, noscapine, phenylpropanolamine, vitamin D, simvastatin, bisacodyl, lactitol, lactulose, magnesium oxide, sodium picosulphate, senna glycosides, benzocaine, lidocaine, tetracaine, almotriptan, eletriptan, naratriptan, rizatriptan, sumatriptan, zolmitriptan, calcium, chromium, copper, iodine, magnesium, manganese, molybdenium, phosphor, selenium, zinc, chloramine, hydrogenperoxide, metronidazole, triamcinolonacetonide, benzethonium chl., cetyl pyrid. chl., chlorhexidine, fluoride, lidocaine, amphotericin, miconazole, nystatin, fish oil, ginkgo biloba, ginseng, ginger, purple coneflower, saw palmetto, cetirizine, levocetirizine, loratadine, diclofenac, flurbiprofen, acrivastine pseudoephedrine, loratadine pseudoephedrine, glucosamine, hyaluronic acid, decapeptide KSL-W, decapeptide KSL, resveratrol, misoprostol, bupropion, ondansetron HCI, esomeprazole, lansoprazole, omeprazole, pantoprazole, rabeprazole, bacteria and the like, loperamide, simethicone, acetylsalicylic acid and others, sucralfate, clotrimazole, fluconazole, itraconazole, ketoconazole, terbinafine, allopurinol, probenecid, atorvastatin, fluvastatin, lovastatin, nicotinic acid, pravastatin, rosuvastatin, simvastatin, pilocarpine, naproxen, alendronate, etidronate, raloxifene, risedronate, benzodiazepines, disulphiram, naltrexone, buprenorphine, codeine, dextropropoxyphene, fentanyl, hydromorphone, ketobemidone, ketoprofen, methadone, morphine, naproxen, nicomorphine, oxycodone, pethidine, tramadol, amoxicillin, ampicillin, azithromycin, ciprofloxacin, clarithromycin, doxycyclin, erythromycin, fusidic acid, lymecycline, metronidazole, moxifloxacin, ofloxacin, oxytetracycline, phenoxymethylpenicillin, rifamycins, roxithromycin, sulphamethizole, tetracycline, trimethoprim, vancomycin, acarbose, glibenclamide, gliclazide, glimepiride, glipizide, insulin, repaglinide, tolbutamide, oseltamivir, aciclovir, famciclovir, penciclovir, valganciclovir, amlopidine, diltiazem, felodipine, nifedipine, verapamil, finasteride, minoxidil, cocaine, buphrenorphin, clonidine, methadone, naltrexone, calcium antagonists, clonidine, ergotamine, β-blockers, aceclofenac, celecoxib, dexiprofen, etodolac, indometacin, ketoprofen, ketorolac, lornoxicam, meloxicam, nabumetone, oiroxicam, parecoxib, phenylbutazone, piroxicam, tiaprofenic acid, tolfenamic acid, aripiprazole, chlorpromazine, chlorprothixene, clozapine, flupentixol, fluphenazine, haloperidol, lithium carbonate, lithium citrate, melperone, penfluridol, periciazine, perphenazine, pimozide, pipamperone, prochlorperazine, risperidone, thioridizin, fluconazole, itraconazole, ketoconazole, voriconazole, opium, benzodiazepines, hydroxine, meprobamate, phenothiazine, aluminiumaminoacetate, esomeprazole, famotidine, magnesium oxide, nizatide, omeprazole, pantoprazole, fluconazole, itraconazole, ketoconazole, metronidazole, amphetamine, atenolol, bisoprolol fumarate, metoprolol, metropolol, pindolol, propranolol, auranofin, and bendazac.

Further examples of useful pharmaceutically active agents or pharmaceutically inactive precursors thereof can include active ingredients selected from the therapeutical groups comprising: Analgesic, Anaesthetic, Antipyretic, Anti-allergic, Anti-arrhythmic, Appetite suppressant, Antifungal, Anti-inflammatory, Broncho dilator, Cardiovascular drugs, Coronary dilator, Cerebral dilator, Peripheral vasodilator, Anti-infective, Psychotropic, Anti-manic, Stimulant, Antihistamine, Laxative, Decongestant, Gastro-intestinal sedative, Sexual dysfunction agent, Disinfectants, Anti-diarrhoeal, Anti-anginal substance, Vasodilator, Anti-hypertensive agent, Vasoconstrictor, Migraine treating agent, Antibiotic, Tranquilizer, Antipsychotic, Anti-tumour drug, Anticoagulant, Antithrombotic agent, Hypnotic, Sedative, Anti-emetic, Anti-nauseant, Anticonvulsant, Neuromuscular agent, Hyper and hypoglycaemic, Thyroid and antithyroid, Diuretic, Antispasmodic, Uterine relaxant, Anti-obesity agent, Anorectic, Spasnolytics, Anabolic agent, Erythropoietic agent, Anti-asthmatic, Expectorant, Cough suppressant, Mucolytic, Anti-uricemic agent, Dental vehicle, Breath freshener, Antacid, Anti-diuretic, Anti-flatulent, Betablocker, Teeth Whitener, Enzyme, Co-enzyme, Protein, Energy booster, Fibre, Probiotics, Prebiotics, NSAID, Anti-tussives, Decongestants, Anti-histamines, Expectorants, Anti-diarrhoeals, Hydrogen antagonists, Proton pump inhibitors, General nonselective CNS depressants, General nonselective CNS stimulants, Selectively CNS function modifying drugs, Antiparkinsonism, Narcotic-analgetics, Analgetic-antipyretics, Psychopharmacological drugs, and Sexual dysfunction agents.

Examples of useful pharmaceutically active agents or pharmaceutically inactive precursors thereof may also include: Casein glyco-macro-peptide (CGMP), Triclosan, Cetyl pyridinium chloride, Domiphen bromide, Quaternary ammonium salts, zinc components, Sanguinarine, Fluorides, Alexidine, Octonidine, EDTA, Aspirin, Acetaminophen, Ibuprofen, Ketoprofen, Diflunisal, Fenoprofen calcium, Naproxen, Tolmetin sodium, Indomethacin, Benzonatate, Caramiphen edisylate, Menthol, Dextromethorphan hydrobromide, Theobromine hydrochloride, Chlophendianol Hydrochloride, Pseudoephedrine Hydrochloride, Phenylephrine, Phenylpropanolamine, Pseudoephedrine sulphate, Brompheniramine maleate, Chlorpheniramine- maleate, Carbinoxamine maleate, Clemastine fumarate, Dexchlorpheniramine maleate, Dephenhydramine hydrochloride, Diphenpyralide hydrochloride, Azatadine maleate, Diphenhydramine citrate, Doxylamine succinate, Promethazine hydrochloride, Pyrilamine maleate, Tripellenamine citrate, Triprolidine hydrochloride, Acrivastine, Loratadine, Brompheniramine, Dexbrompheniamine, Guaifenesin, Ipecac, potassium iodide, Terpin hydrate, Loperamide, Famotidine, Ranitidine, Omeprazole, Lansoprazole, Aliphatic alcohols, Barbiturates, caffeine, strychnine, Picrotoxin, Pentyenetetrazol, Phenyhydantoin, Phenobarbital, Primidone, Carbamazapine, Etoxsuximide, Methsuximide, Phensuximide, Trimethadione, Diazepam, Benzodiazepines, Phenacemide, Pheneturide, Acetazolamide, Sulthiame, bromide, Levodopa, Amantadine, Morphine, Heroin, Hydromorphone, Metopon, Oxymorphone, Levophanol, Codeine, Hydrocodone, Xycodone, Nalorphine, Naloxone, Naltrexone, Salicylates, Phenylbutazone, Indomethacin, Phenacetin, Chlorpromazine, Methotrimeprazine, Haloperidol, Clozapine, Reserpine, Imipramine, Tranylcypromine, Phenelzine, Lithium, Sildenafil citrate, Tadalafil, and Vardenafil CL. For example, eugenol can be used as anaesthetic.

Examples of useful pharmaceutically active agent or pharmaceutically inactive precursor thereof may include active ingredients selected from the groups of ace-inhibitors, antianginal drugs, anti- arrhythmias, anti-asthmatics, anti-cholesterolemics, analgesics, anaesthetics, anticonvulsants, anti-depressants, anti-diabetic agents, anti-diarrhoea preparations, antidotes, anti-histamines, anti-hypertensive drugs, anti-inflammatory agents, anti-lipid agents, anti- manics, anti-nauseants, anti-stroke agents, anti-thyroid preparations, anti-tumour drugs, anti- viral agents, acne drugs, alkaloids, amino acid preparations, anti-tussives, anti- uricemic drugs, anti-viral drugs, anabolic preparations, systemic and non-systemic anti- infective agents, anti-neoplasties, antiparkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocriptine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumour drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of useful pharmaceutically active agents or pharmaceutically inactive precursors thereof contemplated can also include antacids, H2-antagonists, and analgesics. For example, antacid dosages can be prepared using the ingredients calcium carbonate alone or in combination with magnesium hydroxide, and/or aluminium hydroxide. Moreover, antacids can be used in combination with H2-antagonists.

Analgesics include opiates and opiate derivatives, such as Oxycontin™, ibuprofen, aspirin, acetaminophen, and combinations thereof that may optionally include caffeine.

Other useful pharmaceutically active agents or pharmaceutically inactive precursors thereof can include anti-diarrhoeals such as Immodium™ AD, anti-histamines, anti-tussives, decongestants, vitamins, and breath fresheners. Also contemplated for use herein are anxiolytics such as Xanax™; anti-psychotics such as Clozaril™ and Haldol™; non-steroidal anti-inflammatories (NSAID's) such as ibuprofen, naproxen sodium, Voltaren™ and Lodine™, anti-histamines such as Claritin™, Hismanal™, Relafen™, and Tavist™; antiemetics such as Kytril™ and Cesamet™; bronchodilators such as Bentolin™, Proventil™; anti-depressants such as Prozac™, Zoloft™, and Paxil™; anti-migraines such as Imigra™; ACE-inhibitors such as Vasotec™, Capoten™ and Zestril™; anti- Alzheimer's agents, such as Nicergoline™; and CaH-antagonists such as Procardia™, Adalat™, and Calan™.

The popular H2-antagonists which are contemplated for use in the present invention include cimetidine, ranitidine hydrochloride, famotidine, nizatidine, ebrotidine, mifentidine, roxatidine, pisatidine and aceroxatidine.

Active antacid ingredients can include, but are not limited to, the following: aluminium hydroxide, dihydroxyaluminium aminoacetate, aminoacetic acid, aluminium phosphate, dihydroxyaluminium sodium carbonate, bicarbonate, bismuth aluminate, bismuth carbonate, bismuth subcarbonate, bismuth subgallate, bismuth subnitrate, bismuth subsilysilate, calcium phosphate, citrate ion (acid or salt), amino acetic acid, hydrate magnesium aluminate sulphate, magaldrate, magnesium aluminosilicate, magnesium carbonate, magnesium glycinate, magnesium hydroxide, magnesium oxide, magnesium trisilicate, milk solids, aluminium mono-ordibasic calcium phosphate, tricalcium phosphate, potassium bicarbonate, sodium tartrate, sodium bicarbonate, magnesium aluminosilicates, tartaric acids and salts.

In some embodiments, the pharmaceutically active agent or pharmaceutically inactive precursor thereof can be selected from analgesics/anaesthetics such as menthol, phenol, hexylresorcinol, benzocaine, dyclonine hydrochloride, benzyl alcohol, salicyl alcohol, and combinations thereof. In some embodiments, the pharmaceutically active agent or pharmaceutically inactive precursor thereof can be selected from demulcents such as slippery elm bark, pectin, gelatin, and combinations thereof. In some embodiments, the pharmaceutically active agent or pharmaceutically inactive precursor thereof can be selected from antiseptic ingredients such as cetylpyridinium chloride, domiphen bromide, dequalinium chloride, eugenol and combinations thereof.

In some embodiments, the pharmaceutically active agent or pharmaceutically inactive precursor thereof can be selected from antitussive ingredients such as chlophedianol hydrochloride, codeine, codeine phosphate, codeine sulphate, dextromethorphan, dextromethorphan hydrobromide, diphenhydramine citrate, and diphenhydramine hydrochloride, and combinations thereof.

In some embodiments, the pharmaceutically active agent or pharmaceutically inactive precursor thereof can be selected from throat soothing agents such as honey, propolis, aloe vera, glycerine, menthol and combinations thereof. In still other embodiments, the pharmaceutically active agent or pharmaceutically inactive precursor thereof can be selected from cough suppressants. Such cough suppressants can fall into two groups: those that alter the texture or production of phlegm such as mucolytics and expectorants; and those that suppress the coughing reflex such as codeine (narcotic cough suppressants), antihistamines, dextromethorphan and isoproterenol (non-narcotic cough suppressants).

In still other embodiments, the pharmaceutically active agent or pharmaceutically inactive precursor thereof can be an antitussive selected from the group comprising codeine, dextromethorphan, dextrorphan, diphenhydramine, hydrocodone, noscapine, oxycodone, pentoxyverine and combinations thereof. In some embodiments, the pharmaceutically active agent or pharmaceutically inactive precursor thereof can be selected from antihistamines such as acrivastine, azatadine, brompheniramine, chlorpheniramine, clemastine, cyproheptadine, dexbrompheniramine, dimenhydrinate, diphenhydramine, doxylamine, hydroxyzine, meclizine, phenindamine, phenyltoloxamine, promethazine, pyrilamine, tripelennamine, triprolidine and combinations thereof. In some embodiments, the pharmaceutically active agent or pharmaceutically inactive precursor thereof can be selected from non-sedating antihistamines such as astemizole, cetirizine, ebastine, fexofenadine, loratidine, terfenadine, and combinations thereof.

For example, the one or more active ingredient(s) is/are selected from fragrances, flavours, essential oils, insecticide, fungicide, pharmaceutically active agent, or pharmaceutically inactive precursor thereof, e.g. antiseptic and/or anaesthetic, and mixtures thereof.

If the granules comprise at least one active ingredient and/or inactive precursor thereof, the at least one active ingredient and/or inactive precursor thereof is/are preferably present in the granules in an amount from 0.5 to 80 wt.-%, preferably of from 10.0 to 70 wt.-% and most preferably of from 20 to 60 wt.-%, based on the total dry weight of the granules.

It is further to be noted that the granules of the present invention have an improved flowability, compactability as well as mechanical stability compared to granules produced by a method missing homogenizing step b).

Further to this, the granules are ready to use granules in further methods for producing dispersible dosage forms. Such dosage forms are tablets, mini-tables, pellets, capsules, jelly beans or chewing gums comprising these granules.

Further to this, the granules and the above mentioned dosage forms can be used in a nutraceutical product, agricultural product, veterinary product, cosmetic product, home product, food product, packaging product or personal care product, or as excipient in a pharmaceutical product.

It is appreciated that the cosmetic product is preferably a dry cosmetic and/or dry skin care composition, more preferably a dry cosmetic composition. For example, the dry cosmetic composition is a cosmetic powder including eyeshadow, powder makeup, lip powder, face powder, body powder or blusher. According to another embodiment, the cosmetic product is a dry skin care composition. For example, the dry skin care composition may be a skin care powder including shaving powder, body powder, baby powder, foot powder and a deodorant powder. According to yet another embodiment, the dry cosmetic and/or dry skin care composition is a dry cosmetic and skin care composition.

The personal care product is preferably an oral care composition. In one embodiment, the oral care composition is a toothpaste, a toothgel, a toothpowder, a cement, a composition carried out on a mouthstrip or a buccal adhesive patch, a (chewable) tooth tablet, a chewable pastille or a chewable gum, preferably a toothpaste, a toothpowder, a toothpowder or a (chewable) tooth tablet.

Furthermore, the granules and the above mentioned dosage forms can be used in air treatment and in water treatment.

For example, if the granules and the above mentioned dosage forms are used in air treatment, they are especially suitable for absorption and/ or removal of airborne pollutants, such as NOx, $SO_2$, HCl and/or HF species. If the granules and the above mentioned dosage forms are used in water treatment, they are especially suitable for absorption and/or removal of water bourne pollutants, such as heavy metals.

For example, the granules may be used in combination with a surfactant. In particular, the surfactant can be loaded onto the granules.

In one embodiment of the present invention, the surfactant may be selected from anionic surfactants, cationic surfactants, amphoteric surfactants and nonionic surfactants.

Anionic surfactants suitable for use in the present invention can be any anionic surfactant known in the field of water treatment. For example, the anionic surfactant is selected from the group comprising alkane sulphonates, olefin sulphonates, fatty acid ester sulphonates, such as methyl or ethyl ester sulphonates, alkyl aryl sulphonates, alkyl phosphonates, alkyl ether phosphonates, taurates, alkyl ether carboxylates, fatty acids, $C_8$-$C_{22}$ alkyl sulphates, $C_8$-$C_{22}$ alkylbenzene sulphates and salts thereof, $C_8$-$C_{22}$ alkyl alkoxy sulphates and salts thereof, such as sodium lauryl ether sulphate, $C_{12}$-$C_{22}$ methyl ester sulphonates and salts thereof, $C_{12}$-$C_{22}$ alkylbenzene sulphonates and salts thereof, such as sodium dodecylbenzenesulphonate, $C_{12}$-$C_{22}$ fatty acid soaps and salts thereof and mixtures thereof.

Nonionic surfactants suitable for use in the present invention can be any nonionic surfactant known in the field of water treatment. For example, the nonionic surfactant is selected from the group comprising alkyl ethoxylates, such as $C_8$-$C_{22}$ alkyl ethoxylates, $C_6$-$C_{12}$ alkyl phenol alkoxylates, alkylpolysaccharides, alkyl polyglucoside surfactants, glucamides, methylesteralkoxylates, alkoxylated alcohols, such as alkoxylated $C_{12}$-$C_{22}$ alcohols, polyamide emulsifiers, ethylene oxide/propylene oxide block copolymers, fatty alcohols, fatty alcohol alkoxylates, optionally modified fatty alcohol alkoxylates, fatty acid alkoxylates such as fatty acid ethoxylates, optionally modified fatty acid alkoxylates, ethoxylated or propoxylated sorbitan esters, polyhydroxy fatty acid amides, rhamnolipids, glucoselipids, lipopeptides and mixtures thereof.

Cationic surfactants suitable for use in the present invention can be any cationic surfactant known in the field of water treatment. For example, useful cationic surfactants can be selected from fatty amines, quaternary ammonium salts, esterquats, i.e. quaternized fatty acid surfactants, and mixtures thereof.

Amphoteric surfactants suitable for use in the present invention can be any amphoteric surfactant known in the field of water treatment. For example, the amphoteric surfactants can be selected from aliphatic derivatives of secondary or tertiary amines and/or aliphatic derivatives of heterocyclic secondary and tertiary amines in which the aliphatic radical can be a straight- or branched-chain.

In a preferred embodiment of the present invention, the surfactant is selected from the group consisting of alkyl ethoxylates, quaternary ammonium salts, ethylene oxide/propylene oxide block copolymers, fatty acids and salts thereof, alkyl aryl sulphonates, fatty alcohols, aluminum stearate, non-ionic polyamide emulsifiers and mixtures thereof, and most preferably the surfactant is selected from the group consisting of $C_8$-$C_{22}$ alkyl ethoxylates, $C_6$-$C_{12}$ alkyl phenol alkoxylates, tall oil, tallow oil, salts and derivatives thereof, and mixtures of the foregoing.

The term "non-ionic polyamide emulsifier" is understood to refer to non-ionic polyamide emulsifiers based on fatty acids cross-linked with polyamines. Suitable examples of non-ionic polyamide emulsifiers are known to the skilled person.

In one exemplary embodiment, the surfactant is selected from fatty acids and salts thereof. Suitable fatty acids include saturated and unsaturated fatty acids, i.e., saturated and unsaturated carboxylic acids, such as those described above within context of the surface-treatment agents. Suitable salts of fatty acids include calcium and magnesium salts of saturated and unsaturated fatty acids. An illustrative example of a fatty acid suitable for use as a surfactant in the present invention is linoleic acid.

In another exemplary embodiment, the surfactant is selected from tall oil, tallow oil, salts and derivatives thereof. Salts of tall oil and tallow oil suitable for use in the present invention include calcium salts and magnesium salts of tall oil and tallow oil. The tall oil or tallow oil may optionally be derivatized, for example, by oxidation, by derivatization to a tallow amine, e.g. by reaction with a monoamine, diamine or polyamine, by ethoxylation or alkoxylation or by a combination of two or more of the aforementioned derivatization processes. An illustrative example of a tallow oil derivative suitable for use in the present invention is an ethoxylated tallow amine, such as those available from Schärer + Schläpfer under the tradename Aduxol, for example, ethoxylated tallow propylene diamine Aduxol TPA-03 D.

In any of the foregoing embodiments, the term "ethoxylated" or "alkoxylated" is understood to relate to the modification of the respective compound by the addition of an (oligo)ethyleneoxy group $-(CH_2-CH_2-O-)_n$ or alkyleneoxy

group -(Y-O-)$_n$, respectively, wherein Y = alkylene group having from 2 to 6 carbon atoms and n = 1 to 200, preferably 3 to 40.

It is appreciated that the term surfactant in the meaning of the present invention may also comprise a mixture of two or more surfactants.

Mini-tablets or tablets are well known in the art and are of a particle size which is typically used for the products to be prepared.

For example, the mini-tablets or tablets have a weight median particle size $d_{50}$ of from 0.1 to 20.0 mm, preferably 0.2 to 15.0 mm and more preferably from 0.3 to 10.0 mm, as measured according to mechanical sieving.

In view of the above, another aspect of the present invention refers to the use of the granules as defined herein in a nutraceutical product, agricultural product, veterinary product, cosmetic product, preferably in a dry cosmetic and/or dry skin care composition, home product, food product, packaging product, personal care product, preferably in an oral care composition, in air treatment and in water treatment, or as excipient in a pharmaceutical product.

The following examples and tests will illustrate the present invention, but are not intended to limit the invention in any way.

**Brief description of the Figures**

Fig. 1 shows the SEM results for the granules PHM1.
Fig. 2 shows the SEM results for the granules PHM2.
Fig. 3 shows the SEM results for the granules PHM3.
Fig. 4 shows the SEM results for the granules PHM4.
Fig. 5 shows the SEM results for the granules PHM5.

**Examples**

**Measurement methods**

In the following, measurement methods implemented in the examples are described.

**Particle size distribution**

Volume determined median particle size dso(vol) and the volume determined top cut particle size $d_{98}$(vol) as well as the volume particle sizes $d_{90}$(vol) and dio(vol) were evaluated in a wet unit using a Malvern Mastersizer 2000 or 3000 Laser Diffraction System (Malvern Instruments Pic., Great Britain). The dso(vol) or $d_{98}$(vol) value indicates a diameter value such that 50 % or 98 % by volume, respectively, of the particles have a diameter of less than this value. The raw data obtained by the measurement was analyzed using the Mie theory, with a particle refractive index of 1.57 and an absorption index of 0.005. The methods and instruments are known to the skilled person and are commonly used to determine particle size distributions of fillers and pigments. The sample was measured in dry condition without any prior treatment.

The weight determined median particle size $d_{50}$(wt) was measured by the sedimentation method, which is an analysis of sedimentation behaviour in a gravimetric field. The measurement was made with a Sedigraph™ 5120 of Micromeritics Instrument Corporation, USA. The method and the instrument are known to the skilled person and are commonly used to determine particle size distributions of fillers and pigments. The measurement was carried out in an aqueous solution of 0.1 wt.-% $Na_4P_2O_7$. The samples were dispersed using a high speed stirrer and supersonicated.

The processes and instruments are known to the skilled person and are commonly used to determine grain sizes of fillers and pigments.

If not otherwise indicated in the following example section, the volume particle sizes were evaluated in a wet unit using a Malvern Mastersizer 2000 Laser Diffraction System (Malvern Instruments Pic., Great Britain).

**Specific surface area (SSA)**

The BET specific surface area in the meaning of the present invention is defined as the surface area of the particles divided by the mass of the particles. As used herein, the specific surface area is specified in $m^2$/g.

The specific surface area was measured via the BET method according to ISO 9277:2010 using nitrogen, following conditioning of the sample by heating at 110°C, when using disintegrant(s), or at 250°C, when the sample is free of disintegrant(s), for a period of 30 minutes. If the sample was in the form of an aqueous suspension, the sample was filtered within a Büchner funnel, rinsed with deionised water and dried at 110°C in an oven for at least 12 hours prior to such measurement.

**Intra-particle intruded specific pore volume (in cm³/g)**

The specific pore volume was measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 $\mu$m (~ nm). The equilibration time used at each pressure step was 20 seconds. The sample material was sealed in a 5 cm³ chamber powder penetrometer for analysis. The data were corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

The total pore volume seen in the cumulative intrusion data can be separated into two regions with the intrusion data from 214 $\mu$m down to about 1 - 4 $\mu$m showing the coarse packing of the sample between any agglomerate structures contributing strongly. Below these diameters lies the fine inter-particle packing of the particles themselves. If they also have intra-particle pores, then this region appears bi-modal, and by taking the specific pore volume intruded by mercury into pores finer than the modal turning point, i.e. finer than the bi-modal point of inflection, the specific intra-particle pore volume is defined. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the inter-particle pore region and the intra-particle pore region, if present. Knowing the intra-particle pore diameter range it is possible to subtract the remainder inter-particle and inter-agglomerate pore volume from the total pore volume to deliver the desired pore volume of the internal pores alone in terms of the pore volume per unit mass (specific pore volume). The same principle of subtraction, of course, applies for isolating any of the other pore size regions of interest.

**Bulk density**

100 $\pm$ 0.5 g of the respective material were carefully filled through a powder funnel into the 250 mL measuring cylinder and the volume was read off to the nearest 1 mL. The loose bulk density was the calculated according the formula:

$$\text{Loose bulk density [g/mL]} = \text{weighed sample [g]/bulk volume [mL]}$$

and the result was recorded to the nearest 0.01 g/mL.

**Brookfield viscosity**

The Brookfield viscosity is measured by a Brookfield (type RVT) viscometer at 25°C $\pm$ 1°C at 100 rpm after 30 seconds using an appropriate spindle and is specified in mPa·s.

**Weight solids (wt.-%) of a material in suspension**

The weight solids were determined by dividing the weight of the solid material by the total weight of the aqueous suspension. The weight of the solid material is determined by weighing the solid material obtained by evaporating the aqueous phase of the slurry and drying the obtained material to a constant weight.

**Granule stability and granule particle size distribution**

A Malvern Mastersizer 3000 (Malvern Instruments Pic., Great Britain) in combination with Malvern Aero S dry dispersion unit and dry cell was used to determine the particle size distribution of the granules within the fineness range of $d_{50}$ of from 5 to 300 $\mu$m by means of laser diffraction. The methods used are described in the Mastersizer 3000 Basic Guide, Mastersizer 3000 Manual and the Manual for Aero Series Dry dispersion unit available by Malvern Instruments Ltd. Approximately 10 ml of sample was loaded into the Aero S through the corresponding sieve. The sample was measured dry. The results are expressed in V.-% (volume %). The feed rate was done at 0.1 bar, 0.5 bar, and 1.5 bar to show granule stability.

The feed rate of 0.1 bar was used for determining the particle size distribution of the granules.

**Scanning electron microscope (SEM)**

The samples were prepared by diluting 50 to 150 $\mu$l slurry samples with 5 ml water. The amount of slurry sample depends on solids content, mean value of the particle size and particle size distribution. The diluted samples were filtrated by using a 0.8 $\mu$m membrane filter. A finer filter was used when the filtrate is turbid. A doubled-sided conductive adhesive tape was mounted on a SEM stub. This SEM stub was then slightly pressed in the still wet filter cake on the filter. The SEM stub was then sputtered with 8 nm Au. The investigation under the FESEM (Zeiss Sigma VP) was done at 5kV (Au). Subsequently, the prepared samples were examined by using a Sigma VP field emission scanning electron microscope (Carl Zeiss AG, Germany) and a secondary electron detector (SE2) at high vacuum (< $10^{-2}$ Pa).

**Mechanical sieving**

The mechanical sieving was carried out in a vibratory sieve shaker RETSCH AS200 equipped with Easy Sieve Software, sieves according to ISO 3310 incl. sieve pan and a balance (0.1 g). 120 g were used for sieving. The measured sample is made homogeneous to ensure the reproducibility of the sieving at a maximum. The measured sample material was put in the upper test sieve. The sieving was carried out with the following method: sieving time: 3 min / amplitude: 1.0 / interval: 10 s.

**Oil absorption**

The oil absorption was determined in accordance with ISO 787/5.

**2. Materials used**

**Precipitated (or synthetic) hydromagnesite (PHM)**

PHM was obtained by adding 3600 kg fine ground caustic calcined magnesite (MgO) to 40000 liter water having a temperature of 55°C via a top opening into a reactor vessel such that a solids content of ~ 9 wt.-%, based on the total weight of the aqueous suspension, is obtained. The obtained mixture was mixed for about 45 min.
Whilst further mixing the slurry at a temperature between 50 and 55°C, carbonation was started with 4500 - 5000 Nm3/h by using 40% $CO_2$ (+/- 5%) and 80 min.-1 stirrer speed (100%). The temperature of the slurry was allowed to increase during reaction due to the exothermic reaction. The reaction was stopped after conductivity increase, 2.0% $Na_2SO_4$ (d/d PHM) were added under agitation and mixed for 15 minutes before removing the slurry from the vessel.
The slurry obtained (slurry PHM) had a solids content of 16.5 wt.-%, based on the total weight of the slurry, and a Brookfield viscosity of 81 mPa·s.
The characteristics of the precipitated (or synthetic) hydromagnesite are summarized in the following Table 1.

Table 1

| $d_{98}$(vol) [$\mu$m] | $d_{90}$(vol) [$\mu$m] | $d_{50}$(vol) [$\mu$m] | $d_{10}$(vol) [$\mu$m] | Intra particle intruded specific pore volume [$cm^3g^{-1}$] (for the range 0.004 - d* [$\mu$m]) | d* [$\mu$m] |
|---|---|---|---|---|---|
| 61 | 31.7 | 8.95 | 4.65 | 1.461 | 0.93 |

**3. Preparation of granules without homogenizing (Reference)**

**Granules PHM1**

A slurry of the PHM was dried by removing the liquid from the slurry comprising the precipitated (or synthetic) hydromagnesite by means of spray drying using a rotary atomizer of GEA-Niro, Denmark.
The settings used for spray drying are set out in the following Table 2.

Table 2:

| Material | Solids content [wt.-%] | Device | Atomizer speed [%-rpm] | Flow [l/h] |
|---|---|---|---|---|
| Slurry PHM1 | 16.5 | rotary atomizer | 5-9660 | 171 (at 2.3 bar) |

The results for the obtained granules PHM1 are set out in Table 11.

**4. Preparation of granules with homogenizing (inventive)**

**Granules PHM2**

A slurry of the precipitated (or synthetic) hydromagnesite (PHM) was milled in a 25 L vertical stirred media mill of Siegmund Linder containing 15 kg ER102B 0.7/1.4 mm (of company SEPR) at a feed flow of 190 L/h, a tip speed of 5.3 m/s and a specific energy of about 29 kWh/t.

The slurry obtained (slurry PHM2) had a solids content of 17.3 wt.-%, based on the total weight of the slurry.

After milling, the precipitated (or synthetic) hydromagnesite had the characteristics as set out in the following Table 3.

Table 3:

| $d_{98}$(vol) [μm] | $d_{50}$ (vol) [μm] | BET specific surface area [m²/g] |
|---|---|---|
| 50 | 6.75 | 44.3 |

The slurry obtained (slurry PHM2) was then dried by removing the liquid from the slurry comprising the precipitated (or synthetic) hydromagnesite by means of spray drying using a bi-fluid nozzle of GEA-Niro, Denmark.

The settings used for spray drying are set out in the following Table 4.

Table 4:

| Material | Solids content [wt.-%] | Device | Nozzle configuration | Feed flow [l/h] | Pressure [bar] |
|---|---|---|---|---|---|
| Slurry PHM2 | 17.6 | bi-fluid nozzle | 12.9/44/28 | 155 | Air: 1.35 Slurry: 11.6 |

The results for the obtained granules PHM2 are set out in Table 11.

**Granules PHM3**

500 L of the slurry of the precipitated (or synthetic) hydromagnesite (PHM) was pumped once through the homogenizer GEA Ariete NS3055 of GEA Mechanical Equipment Italia S.p.A. at a pressure of 500 bar, a temperature of 50 to 70 °C and a feed flow of 400 L/h at closed screw position and small nozzle.

The slurry obtained (slurry PHM3) had a solids content of 17.6 wt.-%, based on the total weight of the slurry.

After homogenizing, the precipitated (or synthetic) hydromagnesite had the characteristics as set out in the following Table 5.

Table 5:

| $d_{98}$(vol) [μm] | $d_{50}$ (vol) [μm] | BET specific surface area [m²/g] |
|---|---|---|
| 62 | 6.86 | 45.8 |

The slurry obtained (slurry PHM3) was then dried by removing the liquid from the slurry comprising the precipitated (or synthetic) hydromagnesite by means of spray drying using a rotary atomizer of GEA-Niro, Denmark.

The settings used for spray drying are set out in the following Table 6.

Table 6:

| Material | Solids content [wt.-%] | Device | Atomizer speed [%-rpm] | Flow [l/h] |
|---|---|---|---|---|
| Slurry PHM3 | 17.6 | rotary atomizer | 5-9660 | 158 (at 4.4 bar) |

The results for the obtained granules PHM3 are set out in Table 11.

**Granules PHM4**

500 L of the slurry of the precipitated (or synthetic) hydromagnesite (PHM) was pumped once through the homogenizer GEA Ariete NS3055 of GEA Mechanical Equipment Italia S.p.A. at a pressure of 500 bar, a temperature of 50 to 70 °C and a feed flow of 400 L/h at closed screw position and small nozzle.

The slurry obtained (slurry PHM4) had a solids content of 17.6 wt.-%, based on the total weight of the slurry.

After homogenizing, the precipitated (or synthetic) hydromagnesite had the characteristics as set out in the following Table 7.

Table 7:

| $d_{98}$(vol) [μm] | $d_{50}$ (vol) [μm] | BET specific surface area [m²/g] |
|---|---|---|
| 62 | 6.86 | 45.8 |

The slurry obtained (slurry PHM4) was then dried by removing the liquid from the slurry comprising the precipitated (or synthetic) hydromagnesite by means of spray drying using a bi-fluid nozzle of GEA-Niro, Denmark.

The settings used for spray drying are set out in the following Table 8.

Table 8:

| Material | Solids content [wt.-%] | Device | Nozzle configuration | Feed flow [l/h] | Pressure [bar] |
|---|---|---|---|---|---|
| Slurry PHM4 | 17.6 | bi-fluid nozzle | 12.9/44/28 | 155 | Air: 1.35 Slurry: 11.4 |

The results for the obtained granules PHM4 are set out in Table 11.

**Granules PHM5**

500 L of the slurry of the precipitated (or synthetic) hydromagnesite (PHM) was pumped twice through the homogenizer GEA Ariete NS3055 of GEA Mechanical Equipment Italia S.p.A. at a pressure of 500 bar, a temperature of 50 to 70 °C and a feed flow of 400 L/h at closed screw position and small nozzle.

The slurry obtained (slurry PHM5) had a solids content of 17.6 wt.-%, based on the total weight of the slurry.

After two passes, the precipitated (or synthetic) hydromagnesite had the characteristics as set out in the following Table 9.

Table 9:

| $d_{98}$(vol) [μm] | $d_{50}$ (vol) [μm] | BET specific surface area [m²/g] |
|---|---|---|
| 65 | 5.95 | 44.0 |

The slurry obtained (slurry PHM5) was then dried by removing the liquid from the slurry comprising the precipitated (or synthetic) hydromagnesite by means of spray drying using a bi-fluid nozzle of GEA-Niro, Denmark.

The settings used for spray drying are set out in the following Table 10.

Table 10:

| Material | Solids content [wt.-%] | Device | Nozzle configuration | Feed flow [l/h] | Pressure [bar] |
|---|---|---|---|---|---|
| Slurry PHM5 | 17.6 | bi-fluid nozzle | 12.9/44/28 | 155 | Air: 1.35 Slurry: 13.2 |

The results for the obtained granules PHM5 are also set out in the following Table 11.

Table 11:

| Material | Device | $d_{98}$ (vol) [μm] | $d_{90}$ (vol) [μm] | $d_{50}$ (vol) [μm] | $d_{10}$ (vol) [μm] | Bulk density [g/mL] | Intra particle intruded specific pore volume [cm³g⁻¹] (for the range 0.004 - d* [μm]) | d* [μm] |
|---|---|---|---|---|---|---|---|---|
| Granules PHM1 | rotary atomizer | 85.5 | 40.0 | 9.11 | 4.25 | 0.20 | 1.297 | 0.83 |
| Granules PHM2 | bi-fluid nozzle | 104 | 46.8 | 8.90 | 3.49 | 0.29 | 1.457 | 0.83 |
| Granules PHM3 | rotary atomizer | 109 | 69.1 | 21.5 | 3.20 | 0.29 | 1.259 | 0.90 |
| Granules PHM4 | bi-fluid nozzle | 102 | 57.6 | 12.3 | 3.44 | 0.29 | 1.335 | 0.83 |
| Granules PHM5 | bi-fluid nozzle | 128 | 64.2 | 13.6 | 2.93 | 0.32 | 1.185 | 0.83 |

The following table 12 summarizes the granule stability determined by the ratio $d_{50}$ and $d_{10}$ for (0.5 bar) vs. (0.1 bar) and for (1.5 bar) vs. (0.1 bar).

Table 12:

| Material | Device | $d_{50}$(vol)* 0.5 bar vs 0.1 bar | $d_{10}$(vol)* 0.5 bar vs 0.1 bar | $d_{50}$(vol)* 1.5 bar vs 0.1 bar | $d_{10}$(vol)* 1.5 bar vs 0.1 bar |
|---|---|---|---|---|---|
| Granules PHM1 | rotary atomizer | 31.5 | 47.0 | 26.1 | 29.8 |
| Granules PHM2 | bi-fluid nozzle | 43.5 | 47.2 | 16.3 | 22.4 |
| Granules PHM3 | rotary atomizer | 62.5 | 36.2 | 26.4 | 13.9 |
| Granules PHM4 | bi-fluid nozzle | 45.7 | 46.0 | 19.0 | 24.0 |
| Granules PHM5 | bi-fluid nozzle | 54.1 | 39.7 | 24.2 | 18.4 |

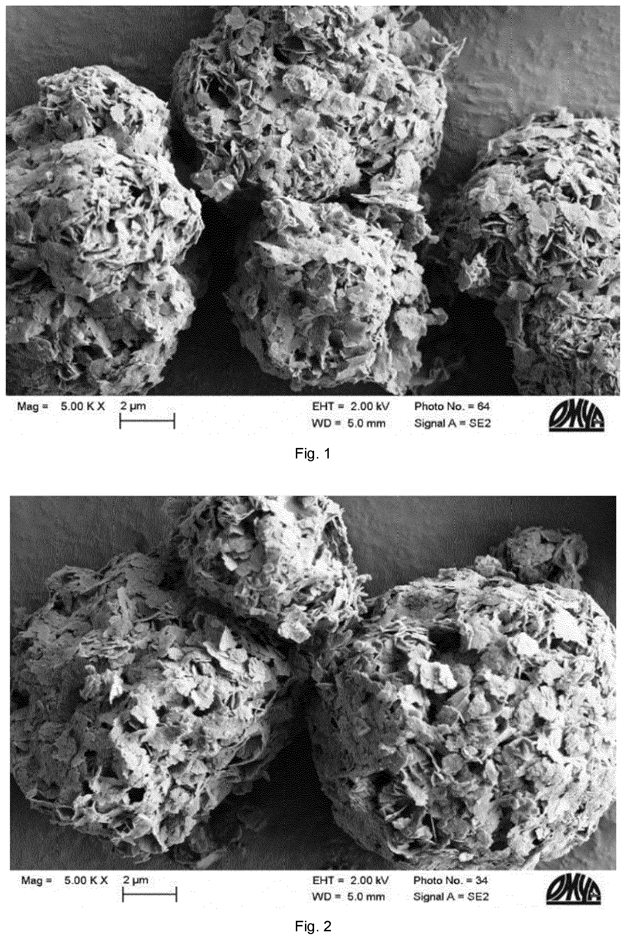

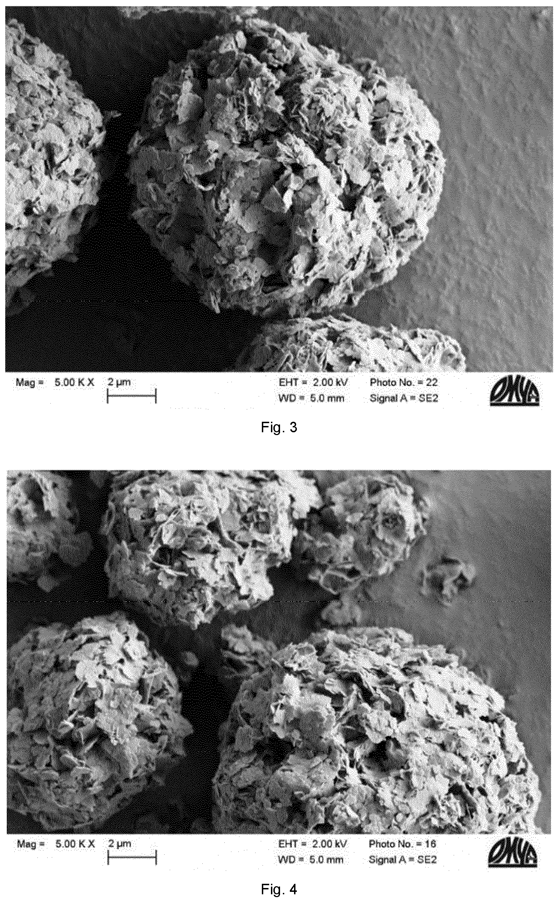

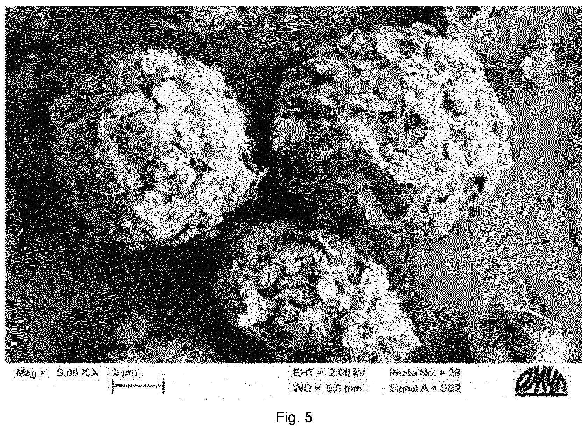

From table 12, it can be gathered that granules prepared by a method comprising a step of homogenizing the aqueous suspension comprising the magnesium ion-comprising material, i.e. Granules PHM2, Granules PHM3, Granules PHM4 and Granules PHM5, are more stable compared to granules obtained by the same method but missing the step of homogenizing the aqueous suspension comprising the magnesium ion-comprising material, i.e. Granules PHM1. It is to be noted that samples after milling as homogenizing step may be slightly inferior in physical data (friability / bulk density) but they are equal in performance. Furthermore, Figures 1 to 5 show a comparison of the SEM results for the granules prepared by a process of the prior art, i.e. Granules PHM1, in comparison to a granules prepared by the process of the present invention, i.e. Granules PHM2, Granules PHM3, Granules PHM4 and Granules PHM5.

The granules prepared by a process of the prior art, i.e. Granules PHM1, as well as the granules prepared according to the present invention, i.e. Granules PHM2, Granules PHM3, Granules PHM4 and Granules PHM5, were further analysed with regard to their oil absorption capacity. The results are shown in the following table 13.

Table 13:

| Material | Device | Oil absorption [%] |
|---|---|---|
| Granules PHM1 | rotary atomizer | 80 |
| Granules PHM2 | bi-fluid nozzle | 91.5 |

(continued)

| Material | Device | Oil absorption [%] |
|---|---|---|
| Granules PHM3 | rotary atomizer | n.d. |
| Granules PHM4 | bi-fluid nozzle | 85.4 |
| Granules PHM5 | bi-fluid nozzle | 91.4 |
| n.d.: not determined | | |

## Claims

1. Method for the production of granules comprising a magnesium ion-comprising material, the method comprising the steps of

    a) providing an aqueous suspension comprising a magnesium ion-comprising material;
    b) homogenizing the aqueous suspension comprising a magnesium ion-comprising material of step a), and
    c) removing the liquid from the aqueous suspension comprising a magnesium ion-comprising material of step b) by means of spray drying for obtaining granules comprising a magnesium ion-comprising material.

2. The method according to claim 1, wherein the magnesium ion-comprising material of step a) is selected from the group comprising a magnesium hydroxide-comprising material, a magnesium carbonate-comprising material, a magnesium oxide-comprising material and mixtures thereof, preferably the magnesium ion-comprising material of step a) is a magnesium carbonate-comprising material selected from the group consisting of dolomite ($CaMg(CO_3)_2$), anhydrous magnesium carbonate or magnesite ($MgCO_3$), hydromagnesite ($Mg_5(CO_3)_4(OH)_2 \cdot 4H_2O$), artinite ($Mg_2(CO_3)(OH)_2 \cdot 3H_2O$), dypingite ($Mg_5(CO_3)_4(OH)_2 \cdot 5H_2O$), giorgiosite ($Mg_5(CO_3)_4(OH)_2 \cdot 5H_2O$), pokrovskite ($Mg_2(CO_3)(OH)_2 \cdot 0.5H_2O$), barringtonite ($MgCO_3 \cdot 2H_2O$), lansfordite ($MgCO_3 \cdot 5H_2O$), dolocarbonate and nesquehonite ($MgCO_3 \cdot 3H_2O$), more preferably the magnesium ion-comprising material of step a) is hydromagnesite, e.g. natural or synthetic hydromagnesite.

3. The method according to claim 1 or 2, wherein the magnesium ion-comprising material of step a) is a surface-reacted magnesium carbonate-comprising material obtained by treating the surface of the magnesium carbonate-comprising material with one or more compound(s) selected from the group consisting of sulphuric acid, phosphoric acid, carbonic acid, carboxylic acids containing up to six carbon atoms, preferably selected from formic acid, acetic acid, propionic acid, lactic acid and mixtures thereof; and di-, and tri-carboxylic acids where the carboxylic acid groups are linked by a chain of 0-4 intermittent carbon atoms, preferably selected from oxalic acid, citric acid, succinic acid, maleic acid, malonic acid, tartaric acid, adipic acid, fumaric acid and mixtures thereof, or a corresponding salt thereof.

4. The method according to any one of claims 1 to 3, wherein the magnesium ion-comprising material of step a) has

    a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and/or
    b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and/or
    c) a BET specific surface area in the range from 10 to 100 $m^2/g$, preferably from 12 to 70 $m^2/g$, and most preferably from 17 to 60 $m^2/g$, measured using nitrogen and the BET method according to ISO 9277:2010; and/or
    d) an intra-particle intruded specific pore volume in the range from 0.9 to 2.3 $cm^3/g$, preferably from 1.2 to 2.1 $cm^3/g$, and most preferably from 1.5 to 2.0 $cm^3/g$, calculated from mercury porosimetry measurement.

5. The method according to any one of claims 1 to 4, wherein the aqueous suspension of step a) has a solids content in the range from 1 to 40 wt.-%, preferably from 5 to 35 wt.-%, and most preferably from 7 to 26 wt.-%, based on the total weight of the aqueous suspension.

6. The method according to any one of claims 1 to 5, wherein at least one disintegrant is added before and/or during and/or after step b), preferably the at least one disintegrant is selected from the group comprising sodium croscarmellose, modified cellulose gums, insoluble cross-linked polyvinylpyrrolidones, starches, modified starches, starch glycolates such as sodium starch glycolate, micro crystalline cellulose, pregelatinized starch, sodium carboxymethyl starch, low-substituted hydroxypropyl cellulose, homopolymers of N-vinyl-2-pyrrolidone, alkyl-, hydroxyalkyl-, carboxyalkyl-cellulose esters, alginic acid, microcrystalline cellulose and its polymorphic forms, ion exchange resins, gums, chitin, chitosan, clays, gellan gum, crosslinked polacrillin copolymers, agar, gelatine, dextrines, acrylic acid polymers, carboxymethylcellulose sodium/calcium, hydroxpropyl methyl cellulose phthalate, shellac, effervescent mixtures such as bicarbonates in combination with one or more acids, e.g. citric acid or tartaric acid, or mixtures thereof.

7.  The method according to claim 6, wherein the at least one disintegrant is added before and/or during and/or after step b) in an amount ranging from 0.3 to 10 wt.-%, preferably from 0.5 to 8 wt.-%, more preferably from 1 to about 5 wt.-% based on the total dry weight of the magnesium ion-comprising material.

8.  The method according to any one of claims 1 to 7, wherein the homogenizing in step b) is carried out once or several times, preferably 1 to 5 times, more preferably 1 to 3 times.

9.  The method according to any one of claims 1 to 8, wherein the homogenizing in step b) is carried out by milling.

10. The method according to any one of claims 1 to 8, wherein the homogenizing in step b) is carried out at

    a) a pressure ranging from 50 to 900 bar, preferably from 100 to 750 bar, and most preferably from 130 to 650 bar, and/or
    b) an initial temperature ranging from 5 to 95°C, preferably from 10 to 80°C, and most preferably from 15 to 60°C.

11. The method according to any one of claims 1 to 10, wherein the spray drying in step c) is carried out at

    a) a feed pressure ranging from 0.1 to 300 bar, preferably from 1 to 100 bar, more preferably from 1 to < 50 bar, and most preferably from 1 to 25 bar, and/or
    b) a temperature measured as inlet temperature ranging from 120 to 950°C, preferably from 175 to 700°C, and most preferably from 180 to 550°C.

12. Granules comprising a magnesium ion-comprising material, wherein the granules have a bulk density ranging from 0.10 to 0.70 g/mL, preferably from 0.12 to 0.65 g/mL, more preferably from 0.20 to 0.60 g/mL and most preferably from 0.15 to 0.50 g/mL.

13. The granules according to claim 12, wherein the granules have

    a) a volume particle size $d_{90}$ of from 15 to 500 $\mu$m, preferably from 20 to 400 $\mu$m, and most preferably from 30 to 250 $\mu$m, as measured dry at 0.1 bar dispersion pressure by laser diffraction, and
    b) a volume median particle size $d_{50}$ of from 5 to 300 $\mu$m, preferably from 8 to 200 $\mu$m, and most preferably from 10 to 150 $\mu$m, as measured dry at 0.1 bar dispersion pressure by laser diffraction, and
    c) a volume particle size $d_{10}$ of from 1 to 100 $\mu$m, preferably from 2 to 70 $\mu$m, and most preferably from 4 to 50 $\mu$m, as measured dry at 0.1 bar dispersion pressure by laser diffraction, and/or
    d) a BET specific surface area in the range from 20 to 90 m$^2$/g, preferably from 30 to 80 m$^2$/g, and most preferably from 40 to 70 m$^2$/g, measured using nitrogen and the BET method according to ISO 9277:2010, and/or
    e) a spherical shape.

14. The granules according to claim 12 or 13, wherein the granules comprise particles of a magnesium ion-comprising material having

    a) a volume median particle size $d_{50}$ in the range from 1 to 75 $\mu$m, preferably from 1.2 to 50 $\mu$m, more preferably from 1.5 to 30 $\mu$m, even more preferably from 1.7 to 15 $\mu$m and most preferably from 1.9 to 10 $\mu$m, as determined by laser diffraction, and/or
    b) a volume top cut particle size $d_{98}$ in the range from 2 to 150 $\mu$m, preferably from 4 to 100 $\mu$m, more preferably from 6 to 80 $\mu$m, even more preferably from 8 to 60 $\mu$m and most preferably from 10 to 40 $\mu$m, as determined by laser diffraction; and/or
    c) a BET specific surface area in the range from 10 to 100 m$^2$/g, preferably from 12 to 70 m$^2$/g, and most preferably from 17 to 60 m$^2$/g, measured using nitrogen and the BET method according to ISO 9277:2010; and/or

d) an intra-particle intruded specific pore volume in the range from 0.9 to 2.3 $cm^3$/g, preferably from 1.2 to 2.1 $cm^3$/g, and most preferably from 1.5 to 2.0 $cm^3$/g, calculated from mercury porosimetry measurement.

15. The granules according to any one of claims 12 to 14, wherein the granules are obtained by a method according to any one of claims 1 to 11.

16. Use of granules according to claims 12 to 15 in a nutraceutical product, agricultural product, veterinary product, cosmetic product, preferably in a dry cosmetic and/or dry skin care composition, home product, food product, packaging product, personal care product, preferably in an oral care composition, in air treatment and in water treatment, or as excipient in a pharmaceutical product.

Fig. 1

Fig. 2

Mag = 5.00 K X    2 µm      EHT = 2.00 kV    Photo No. = 22
                            WD = 5.0 mm     Signal A = SE2

Fig. 3

Mag = 5.00 K X    2 µm      EHT = 2.00 kV    Photo No. = 16
                            WD = 5.0 mm     Signal A = SE2

Fig. 4

Fig. 5

Europäisches Patentamt

European Patent Office

Office européen des brevets

# EUROPEAN SEARCH REPORT

Application Number

EP 21 19 3840

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TEKENAKA HIDEO ET AL: "Spray Drying Agglomeration. I. Physiochemical Properties of Agglomerated Synthetic Alumunium Silicate or Magnesium Carbonate", CHEM. PHARM. BULL., vol. 19, no. 6, 1 January 1971 (1971-01-01), pages 1234-1244, XP55904779, Retrieved from the Internet: URL:https://www.jstage.jst.go.jp/article/c pb1958/19/6/19_6_1234/_pdf> * page 1234-1235 "Experimental" * * page 1240; table IV * | 1-16 | INV. C01F5/24 C09C1/02 A23L29/00 A61K8/00 A61K33/10 |
| X | PILARSKA AGNIESZKA ET AL: "The Influence of Spray Drying on the Dispersive and Physicochemical Properties of Magnesium Oxide", DRYING TECHNOLOGY., vol. 29, no. 10, 1 August 2011 (2011-08-01), pages 1210-1218, XP55904751, US ISSN: 0737-3937, DOI: 10.1080/07373937.2011.579698 Retrieved from the Internet: URL:https://www.tandfonline.com/doi/pdf/10 .1080/07373937.2011.579698?needAccess=true > * page 1211-1212 "Procedures and Methods" * * table 1 * | 1-16 | |
| X | JP 4 944466 B2 (UBE MAT IND LTD) 30 May 2012 (2012-05-30) * example * | 1-16 | TECHNICAL FIELDS SEARCHED (IPC) C01F C09C A23L A61K A61Q |

-/--

The present search report has been drawn up for all claims

| Place of search | Date of completion of the search | Examiner |
|---|---|---|
| Munich | 1 April 2022 | Besana, Sonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

**EP 21 19 3840**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 733 154 A1 (OMYA INT AG [CH]) 4 November 2020 (2020-11-04) * paragraphs [0029] – [0031] * ----- | 1-11 | |
| | | | **TECHNICAL FIELDS SEARCHED (IPC)** |

The present search report has been drawn up for all claims

| Place of search | Date of completion of the search | Examiner |
|---|---|---|
| Munich | 1 April 2022 | Besana, Sonia |

CATEGORY OF CITED DOCUMENTS

X : particularly relevant if taken alone
Y : particularly relevant if combined with another
      document of the same category
A : technological background
O : non-written disclosure
P : intermediate document

T : theory or principle underlying the invention
E : earlier patent document, but published on, or
      after the filing date
D : document cited in the application
L : document cited for other reasons
............................................................................................
& : member of the same patent family, corresponding
      document

EPO FORM 1503 03.82 (P04C01)

## ANNEX TO THE EUROPEAN SEARCH REPORT
## ON EUROPEAN PATENT APPLICATION NO.

EP 21 19 3840

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2022

| Patent document cited in search report | | Publication date | Patent family member(s) | | Publication date |
|---|---|---|---|---|---|
| JP 4944466 | B2 | 30-05-2012 | JP | 4944466 B2 | 30-05-2012 |
| | | | JP | 2007254193 A | 04-10-2007 |
| EP 3733154 | A1 | 04-11-2020 | AR | 118804 A1 | 03-11-2021 |
| | | | AU | 2020267790 A1 | 30-09-2021 |
| | | | CA | 3128822 A1 | 12-11-2020 |
| | | | CN | 113747874 A | 03-12-2021 |
| | | | CO | 2021014841 A2 | 19-11-2021 |
| | | | EP | 3733154 A1 | 04-11-2020 |
| | | | EP | 3962438 A1 | 09-03-2022 |
| | | | KR | 20220005059 A | 12-01-2022 |
| | | | SG | 11202109641T A | 28-10-2021 |
| | | | TW | 202106277 A | 16-02-2021 |
| | | | WO | 2020225064 A1 | 12-11-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3733785 A1
- EP 3517502 A1
- US 1361324 A
- US 935418 A
- GB 548197 A
- GB 544907 A
- US 5979461 A
- EP 0526121 A

- GB 594262 A
- US 2007194276 A
- WO 2013139957 A1
- WO 2015039994 A1
- US 20030157213 A1
- US 20030206993 A
- US 20030099741 A1
- US 4985459 A, Sunshine

**Non-patent literature cited in the description**

- **GANE, P.A.C. ; KETTLE, J.P. ; MATTHEWS, G.P. ; RIDGWAY, C.J.** Void Space Structure of Compressible Polymer Spheres and Consolidated Calcium Carbonate Paper-Coating Formulations. *Industrial and Engineering Chemistry Research,* 1996, vol. 35 (5), 1753-1764